# FASCICULE DE BREVET EUROPEEN

(11) **EP 0 680 967 B1**
(45) Date de publication et mention de la délivrance du brevet: **14.10.1998**
(21) Numéro de dépôt: 95400987.4
(22) Date de dépôt: 02.05.1995
(51) Int. Cl.: C07H 17/08, A61K 31/70

(54) **Dérivés de l'érythromycine, leur procédé de préparation et leur application comme médicaments**
Erythromycinderivate, ihre Verfahren zur Herstellung und ihre Verwendung als Arzneimittel
Erythromycin derivatives, their process of preparation and their use as medicaments

(30) Priorité: 03.05.1994 FR 9405368
(43) Date de publication de la demande: 08.11.1995
(73) Titulaire: HOECHST MARION ROUSSEL, 92800 Puteaux (FR)
(72) Inventeur: Agouridas, Constantin, F-94130 Nogent sur Marne (FR); Chantot, Jean-François, F-94130 Nogent sur Marne (FR); Denis, Alexis, F-75011 Paris (FR); Gouin d'Ambrieres, Solange, F-75020 Paris (FR); Le Martret, Odile, F-75016 Paris (FR)
(74) Mandataire: Vieillefosse, Jean-Claude

(56) Documents cités:
- EP-A- 0 487 411
- EP-A- 0 596 802
- EP-A- 0 606 024
- EP-A- 0 606 062
- FR-A- 2 692 579
- JOURNAL OF ORGANIC CHEMISTRY., vol.53, no.10, 1988, EASTON US pages 2340 - 2345 W.R.BAKER ET AL. 'Modification of Macrolide Antibiotics. Synthesis of 11-deo xy-11-(carboxyamino)-6-O-methylerythromyci n A 11,12-(cyclic esters) via an Intramolecular Michael Reaction of O-Carbamates with an alpha,beta-Unsaturated Ketone.'

## Description

La présente invention concerne de nouveaux dérivés de l'érythromycine, leur procédé de préparation et leur application comme médicaments.

L'invention a pour objet les composés de formule (I) : 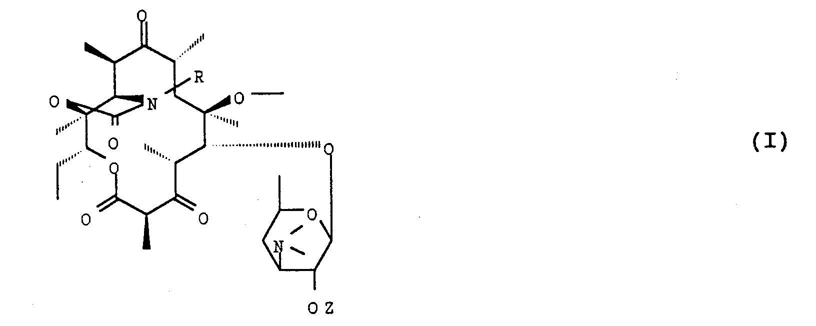 dans laquelle R représente un radical -(CH₂)ₙAr, dans lequel n représente le nombre 3, 4 ou 5 et Ar représente un radical hétérocyclique, portant éventuellement un ou plusieurs substituants, choisi dans le groupe des radicaux : 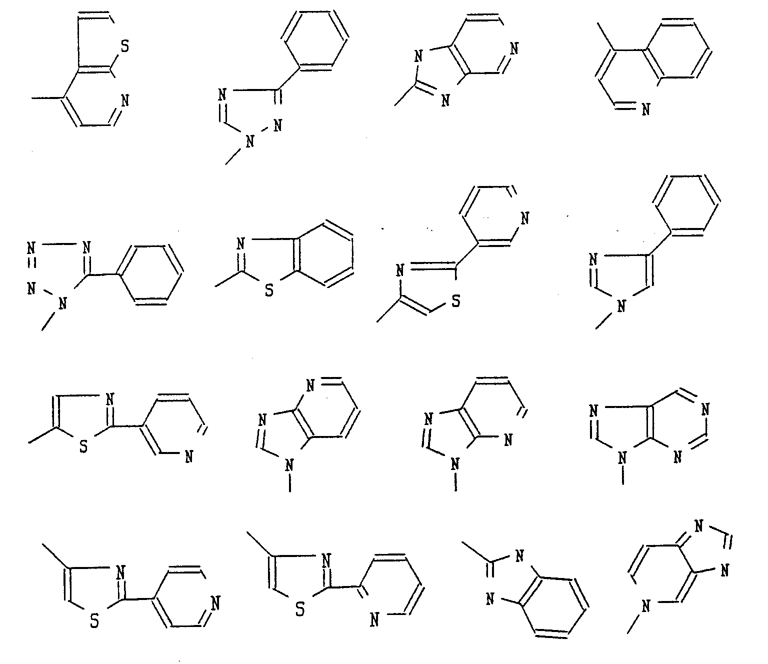 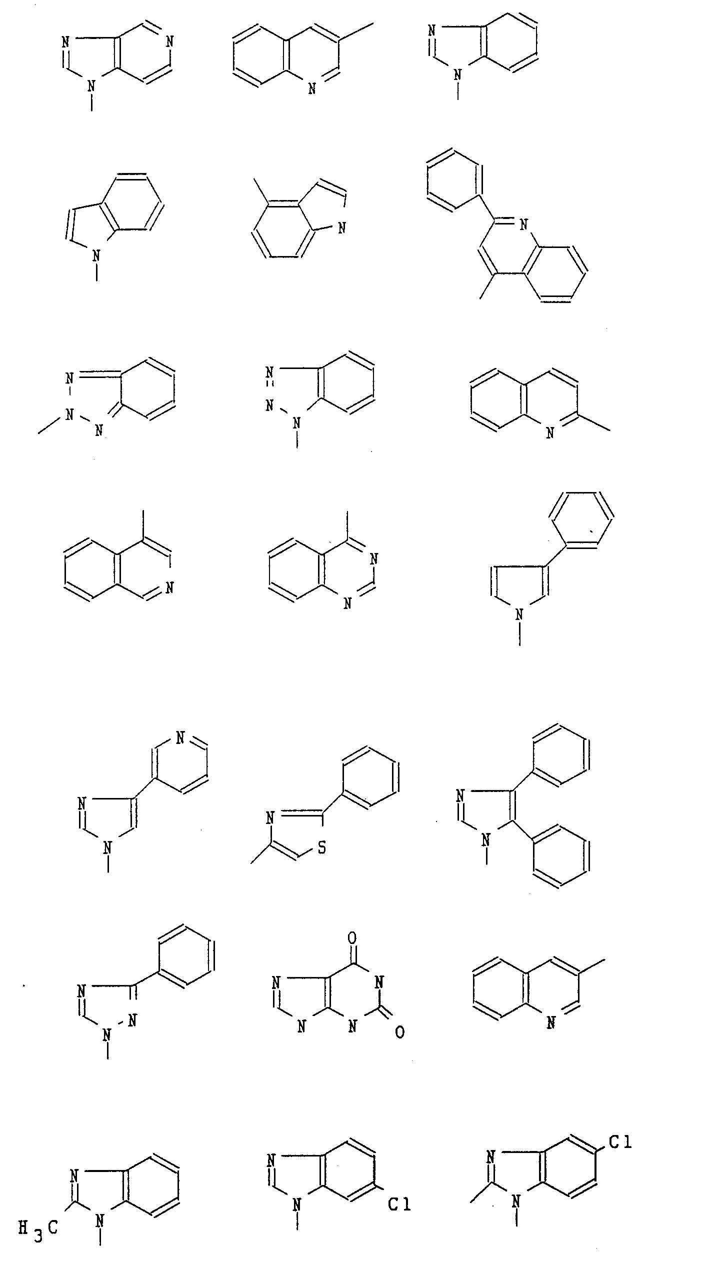 et Z représente un atome d'hydrogène ou le reste d'un acide, ainsi que leurs sels d'addition avec les acides, à l'exception des composés suivants :
11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(1H-indol-4-yl)butyl)imino) érythromycine
11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-quinoléinyl) butyl) imino) érythromycine
11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl (4-(1H-benzimidazol-1-yl) butyl) imino) érythromycine.

Comme exemple de sels d'addition des présents dérivés avec les acides minéraux ou organiques, on peut citer les sels formés avec les acides acétique, propionique, trifluoroacétique, maléique, tartrique, méthanesulfonique, benzènesulfonique, p-toluènesulfonique, chlorhydrique, bromhydrique, iodhydrique, sulfurique, phosphorique et spécialement les acides stéarique, éthylsuccinique ou laurylsulfurique.

Le radical hétérocyclique peut être substitué par un ou plusieurs radicaux choisis dans le groupe constitué par les radicaux carboxyle libre, salifié, estérifié et amidifié, le radical hydroxyle, les atomes d'halogène, les radicaux NO₂, C≡N, les radicaux alkyle, linéaire, ramifié ou cyclique , alkényle et alkynyle, linéaire ou ramifié, O-alkyle, O-alkényle et O-alkynyle, S-alkyle, S-alkényle ou S-alkynyle et N-alkyle, N-alkényle et N-alkynyle, renfermant jusqu'à 12 atomes de carbone éventuellement substitués par un ou plusieurs atomes d'halogène, le radical 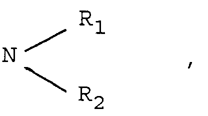 R₁ et R₂ identiques ou différents, représentant un atome d'hydrogène ou un radical alkyle renfermant jusqu'à 12 atomes de carbone, les radicaux aryle, O-aryle ou S-aryle carbocycliques ou aryle, O-aryle ou S-aryle hétérocycliques comportant un ou plusieurs hétéroatomes, éventuellement substitués par un ou plusieurs des substituants mentionnés ci-dessus et le radical 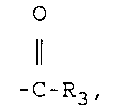 R₃ représentant un radical alkyle renfermant jusqu'à 12 atomes de carbone, ou un radical aryle carbocyclique ou hétérocyclique éventuellement substitué.

Lorsque le radical hétérocyclique comporte plusieurs cycles (reliés entre eux, ou condensés) le ou les substituants peuvent se trouver sur l'un et/ou l'autre des cycles hétérocyliques ou carbocycliques ; c'est ainsi par exemple que si un noyau hétérocyclique est relié ou condensé à un radical aryle, le noyau hétérocyclique et le noyau aryle peuvent tous deux porter un ou plusieurs substituants.

Le radical aryle est de préférence un radical phényle ou naphtyle,
- le radical alkyle, alkényle ou alkynyle est de préférence un radical méthyle, éthyle, propyle, isopropyle, n-butyle, isobutyle, terbutyle, décyle ou dodécyle, vinyle, allyle, éthynyle, propynyle, propargyle, cyclobutyle, cyclopentyle ou cyclohexyle,
- l'halogène est de préférence le fluor, le chlore, ou le brome,
- le radical alkyle substitué par un atome d'halogène est de préférence un radical CHCl₂, CHBr₂, CHF₂, CCl₃, CBr₃, CF₃, CH₂CF₃, CH₂CH₂CCl₃, CH₂CH₂CF₃,
- le reste d'acide carboxylique est de préférence le reste acétyle, propionyle, butyryle, isobutyryle, n-valéryle, isovaléryle, tert-valéryle et pivalyle.

L'invention a plus particulièrement pour objet les composés de formule (I), dans lesquels Z représente un atome d'hydrogène et les composés de formule (I) dans lesquels n représente le nombre 4.

L'invention a tout spécialement pour objet les composés de formule (I) dans lesquels Ar représente un radical : 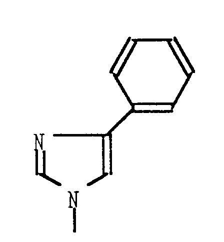 éventuellement substitué, ainsi que les composés de formule (I) dans lesquels R représente le radical : 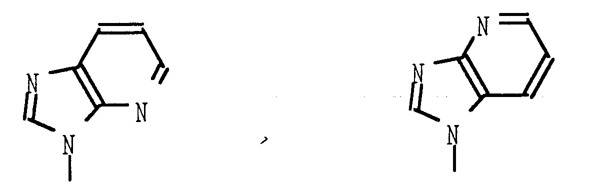 éventuellement substitué, ainsi que les composés de formule (I) dans lesquels Ar représente un radical : 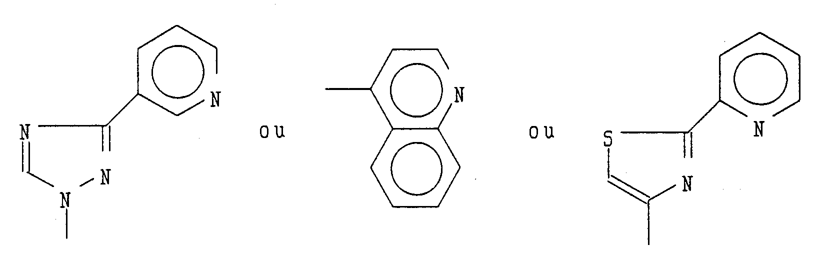 éventuellement substitué et tout particulièrement les composés de formule (I) dans lesquels Ar représente un radical 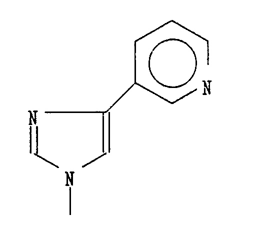 éventuellement substitué.

L'invention a plus particulièrement pour objet les composés de formule (I) dont la préparation est donnée ci-après dans la partie expérimentale. Parmi les composés préférés de l'invention, on peut citer les composés suivants :
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-phényl 1H-imidazol-1-yl) butyl) imino)) érythromycine,
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(3H-imidazo(4,5-b)pyridin-3-yl) butyl) imino)) érythromycine,
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(1H-imidazo(4,5-b)pyridin-1-yl) butyl) imino)) érythromycine,
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-(4-chlorophényl) 1H-imidazol-1-yl) butyl) imino)) érythromycine,
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-(2-méthoxyphényl) 1H-imidazol-1-yl) butyl) imino)) érythromycine,
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-(4-fluorophényl) 1H-imidazol-1-yl) butyl) imino)) érythromycine,
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(7-méthoxy 4-quinoléinyl) butyl) imino)) érythromycine,
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(2-(2-pyridinyl) 4-thiazolyl) butyl) imino)) érythromycine,
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(3-(3-pyridinyl) 1H-1,2,4-triazol-1-yl) butyl) imino)) érythromycine, et tout particulièrement le
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-(3-pyridinyl) 1H-imidazol-1-yl) butyl) imino)) érythromycine.

Les produits de formule générale (I) possèdent une très bonne activité antibiotique sur les bactéries gram ⊕ telles que les staphylocoques, les streptocoques, les pneumocoques.

Les composés de l'invention peuvent donc être utilisés comme médicaments dans le traitement des infections à germes sensibles et notamment, dans celui des staphylococcies, telles que les septicémies à staphylocoques, staphylococcies malignes de la face ou cutanées, pyodermites, plaies septiques ou suppurantes, furoncles, anthrax, phlegmons, érysipèles et acné, staphylococcies telles que les angines aiguës primitives ou post-grippales, bronchopneumonies, suppurations pulmonaires, les streptococcies telles que les angines aiguës, les otites, les sinusites, la scarlatine, les pneumococcies telles que les pneumonies, les bronchites ; la brucellose, la diphtérie, la gonococcie.

Les produits de la présente invention sont également actifs contre les infections dues à des germes comme Haemophilus influenzae, Rickettsies, Mycoplasma pneumoniae, Chlamydia, Legionella, Ureaplasma, Toxoplasma ou à des germes du genre Mycobactérium, Listeria, Meningocoques et Campylobacter.

La présente invention a donc également pour objet, à titre de médicaments et, notamment de médicaments antibiotiques, les produits de formule (I) tels que définis ci-dessus, ainsi que leurs sels d'addition avec les acides minéraux ou organiques pharmaceutiquement acceptables.

L'invention a plus particulièrement pour objet, à titre de médicaments et, notamment de médicaments antibiotiques, les produits préférés de formule (I) définis précédemment à savoir les produits des exemples 1, 2, 3 et 28 à 34, ainsi que leurs sels pharmaceutiquement acceptables.

L'invention a également pour objet les compositions pharmaceutiques renfermant comme principe actif au moins un des médicaments définis ci-dessus.

Ces compositions peuvent être administrées par voie buccale, rectale, parentérale ou par voie locale en application topique sur la peau et les muqueuses, mais la voie d'administration préférée est la voie buccale.

Elles peuvent être solides ou liquides et se présenter sous les formes pharmaceutiques couramment utilisées en médecine humaine, comme par exemple, les comprimés, simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent également se présenter sous forme d'une poudre destinée à être dissoute extemporanément dans un véhicule approprié, par exemple de l'eau stérile apyrogène.

La dose administrée est variable selon l'affection traitée, le sujet en cause, la voie d'administration et le produit considéré. Elle peut être, par exemple, comprise entre 50 mg et 300 mg par jour par voie orale, chez l'adulte pour le produit de l'exemple 1 ou de l'exemple 2.

L'invention a également pour objet un procédé de préparation des composés de formule (I), caractérisé en ce que l'on soumet un composé de formule (II) : 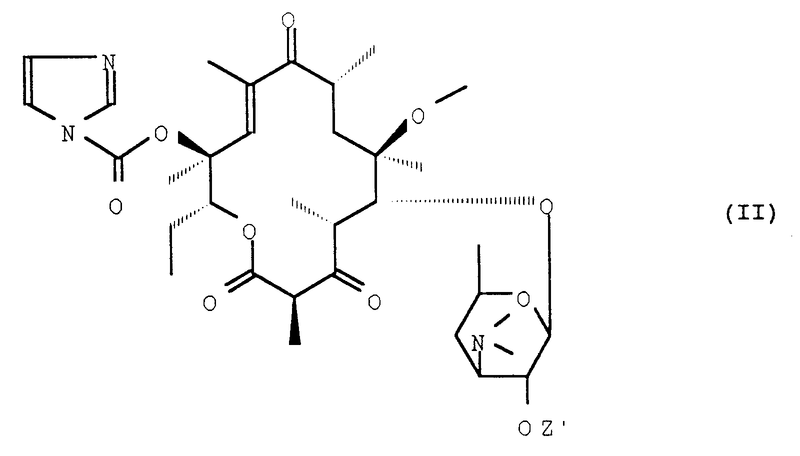 dans laquelle Z' représente le reste d'un acide, à l'action d'un composé de formule (III) :

RNH₂ (III)

dans laquelle R est défini comme précédemment, pour obtenir le composé de formule (I_{A}) : 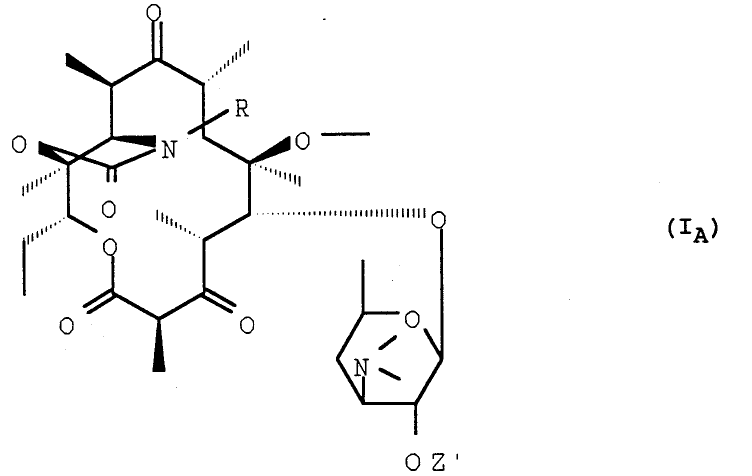 dans lesquels R et Z' conservent leur signification précédente, puis soumet le cas échéant, le composé de formule (I_{A}) à l'action d'un agent de libération de la fonction hydroxyle en 2' et/ou le cas échéant, à l'action d'un acide pour en former le sel,
- la réaction du composé de formule (II) avec le composé de formule (III) a lieu au sein d'un solvant tel que par exemple l'acétonitrile, le diméthylformamide ou encore le tétrahydrofuranne, le diméthoxy éthane ou le diméthylsulfoxyde,
- l'hydrolyse de la fonction ester en 2' est réalisée à l'aide du méthanol ou de l'acide chlorhydrique aqueux,
- la salification est réalisée au moyen d'acides selon les procédés classiques.

Les composés de formule (II) utilisés comme produits de départ sont décrits et revendiqués dans la demande de brevet européen 0 596 802.

Les composés de formule RNH₂ sont des produits connus d'une façon générale, toutefois les composés précis utilisés pour la préparation des produits des exemples sont nouveaux et sont en eux-mêmes un objet de l'invention, leur préparation est donnée ci-après.

Les composés de formule (III) :

RNH₂ (III)

peuvent par exemple être préparés selon les procédés décrits dans J. Med. Chem (1982) vol. 25 p. 947 et suivantes, Tetrahedron Letters vol. 32, n° 14, p. 1699-1702, (1991) ; J. Org. Chem. 54 (18) 4298, 301 (1989) ; J. Org. Chem. 28 (101) 2589 91 (1963) ou le brevet allemand 3 406 416. ; J. Org. Chem. 6-895-901 (1941) ou Synth. Commun 17 (14) 1741-8 (1987).

L'invention a particulièrement pour objet les amines de formule (III) telle que définie ci-dessus dont la préparation est détaillée ci-après.

L'invention a tout particulièrement pour objet :
- la 4-phényl-1H-imidazole 1-butanamine,
- la 3H-imidazo(4,5-b)-pyridine 3-butanamine,
- la 1H-imidazo(4,5-b)-pyridine 3-butanamine,
- la 2-phényl-4-quinoleinebutanamine,
- la 1H-benzotriazole 1-butanamine,
- la 2H-benzotriazole 2-butanamine,
- la 1-méthyl 1H-imidazo(4,5-c)-pyridine 2-butanamine,
- la 3-méthyl 3H-imidazo(4,5-c)-pyridine 2-butanamine,
- la 5-chloro 1H-benzimidazole 1-butanamine,
- la 7-méthoxy 4-quinolèinebutanamine,
- la 1H-imidazo(4,5-c) pyridine 1-butanamine,
- la 9H-purine 9-butanamine,
- la 1-méthyl 1H-indole 4-butanamine,
- la 3-phényl 1H-1,2,4-triazole 1-butanamine (chlorhydrate),
- la 5-phényl 1H-tétrazole 1-butanamine (chlorhydrate),
- la 2-benzothiazolebutanamine,
- la 4-(thiéno(2,3-b) pyridine 4-yl butanamine,
- la 5,6-diméthyl 1H-benzimidazole 1-butanamine,
- la 3-quinoléine butanamine,
- la 2-quinoléine butanamine,
- la 5H-imidazo [4,5-c] pyridine 5-butanamine,
- la 1-méthyl 1H-benzimidazol 2-butanamine,
- la 6-chloro 1H-benzimidazol 2-butanamine,
- la 2-méthyl 1H-benzimidazol 2-butanamine,
- la 4-(4-chlorophényl) 1H-imidazol 1-butanamine,
- la 2-(3-pyridinyl) thiazol 5-butanamine,
- la 7-méthoxyquinoléine 4-butanamine,
- la 4-(4-fluorophényl) 1H-imidazol 1-butanamine,
- la 4-(2-méthoxyphényl) 1H-imidazol 1-butanamine,
- la 3-(3-pyridinyl) 1H 1,2,4-triazol 1-butanamine,
- la 4-(3-pyridinyl) 1H-imidazol 1-butanamine,
- la 2-(2-pyridinyl) thiazol 4-butanamine,
- la 2-phénylthiazol 4-butanamine,
- la 4-(4-méthoxyphényl) 1H-imidazol 1-butanamine,
- l'isoquinoléine 4-butanamine,
- la quinazoline 4-butanamine,
- la 4,5-diphényl 1H-imidazol 1-butanamine,
- la 4-(3-méthoxyphényl) 1H-imidazol 1-butanamine,
- la 4-(4-(trifluorométhoxy) phényl) 1H-imidazol 1-butanamine,
- la 1,2,3,6-tétrahydro 1,3-diméthyl 2,6-dioxo 7H-purine 7-butanamine,
- la 2-(4-pyridinyl) thiazol 4-butanamine,
- la 1H-indol 1-butanamine,
- la 2-(3-pyridinyl) thiazol 4-butanamine
ainsi que leurs sels d'addition avec les acides.

### Les exemples suivants illustrent l'invention.

### EXEMPLE 1 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-phényl 1H-imidazol-1-yl) butyl) imino)) érythromycine

On porte à 63°C un mélange de 0,705 g du produit de la 2'-acétate de 11-déoxy 10,11-didéhydro 3-de(2,6-didéoxy 3-C-méthyl 3-O-méthyl alpha-L-ribohexopyranosyl) oxy) 12-O-((1H-imidazol-1-yl) carbonyl) 6-O-méthyl 3-oxo érythromycine préparé comme indiqué à l'exemple 1C de la demande de brevet européen EP. 0 596 802 dans 3 ml d'acétonitrile à 10 % d'eau, et 1,08 g de 4-(4-phényl 1H-imidazol-1-yl) butanamine. On maintient le mélange réactionnel à cette température pendant 5 heures. On laisse revenir à la température ambiante, verse le mélange réactionnel sur une solution de phosphate acide de sodium, extrait à l'acétate d'éthyle. On lave les phases organiques à l'eau, les sèche, les filtre et les concentre. On obtient 1,5 g d'un produit auquel on ajoute 210 ml de méthanol. On maintient sous agitation pendant 16 heures, sous une atmosphère d'azote, à la température ambiante. On concentre et obtient 1,4 g de produit que l'on purifie par chromatographie sur silice éluant CH₂Cl₂ - MeOH - NH₄OH (93-7-0,4). On concentre et obtient 0,305 g du produit recherché brut, que l'on recristallise dans l'éther isopropylique, lave, sèche à 50°C sous pression réduite. On obtient ainsi 0,267 g du produit recherché fondant à 222°C-231°C.
RMN CDCl₃ ppm
α_{D} = +18° (c = 0,9 CHCl₃)
   0,84 (t) : CH₃-CH₂ ; 1,01 (d)-1,17 (d)-1,24 (d) : les CH₃-CH; 1,30 (d)-1,38 (d), 1,34 à 1,47 : 6 et 12-Me ; 2,27 (s) : N(Me)₂ ; 2,45 (-) : H'₃ ; 2,61 (m) : H₈ ; 2,63 (s) : 6-OMe ; 3,04 (-) : H₄ ; 3,13 (q) : H₁₀ ; 3,18 (dd) : H'₂ ; 3,53 (-) : H'₅ ; 3,56 (s) : H₁₁ ; 3,67 (-), 3,75 (-) : les 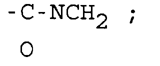 3,87 (q) : H₂ ; 3,99 (t) : CH₂NC ; 4,23 (d) : H₅ ; 4,27 (d) : H'₁ ; 4,94 (dd) : H₁₃ ; 7,26 (s) : H''₅ ; 7,5 (s) : H''₂ ; 7,20 : H en para ; 7,35 : H en méta ; 7,76 : H en ortho.

### PREPARATION 1 : 4-phényl-1H-imidazole-1-butanamine

### Stade A : 2-(4-(4-phényl 1H-imidazol 1-yl) butyl 1H-iso indole-1,3(2H)dione

On introduit goutte à goutte en 1 h 30, une solution renfermant 5,05 g de 4-phényl 1H-imidazole dans 25 cm³ de diméthylformamide dans un mélange de 7 cm³ de diméthylformamide et 2,02 g d'hydrure de sodium. On introduit ensuite 10,86 g de N-4-bromobutylphtalimide en solution dans 25 cm³ de diméthylformamide. On porte la solution obtenue à 70°C pendant 1 h 30 environ. On laisse revenir à la température ambiante, concentre la solution obtenue, reprend à l'eau, extrait à l'acétate d'éthyle. On lave les phases organiques à l'eau, les sèche, les filtre et les concentre. On obtient 15 g de produit que l'on recristallise dans l'acétate d'éthyle. On essore le produit obtenu, le lave à l'acétate d'éthyle et le sèche sous pression réduite à 50°C. On obtient 5,5 g de produit recherché, fondant à 130 ∼ 132°C.
RMN CDCl₃ ppm
   1,75 (m) (2H)-1,86 (m) (2H) : CH₂ centraux ; 3,74 (t) : 2H ; 4,03 : 2H ; 7,22 (t) : 2H H₄ ; 7,26 (m) : 1H H'₃ ; 7,36 (t) : 2H H₃ et H₅ ; 7,56 (d) : H'₅ ; ∼ 7,73 (m) : 4H ; ∼ 7,86 (m) : H₂ et H₆.

### Stade B : 4-phényl-1H-imidazole-1-butanamine

On maintient au reflux pendant 8 heures un mélange de 3,45 g de produit obtenu au stade A, 100 ml d'éthanol et 0,97 ml d'hydrate d'hydrazine. On concentre le mélange réactionnel, ajoute environ 50 ml de soude 2N, extrait à l'acétate d'éthyle. On lave les phases organiques à l'aide de soude 2N, puis de chlorure de sodium. On sèche, filtre et concentre. On obtient 2,21 g de produit recherché.
RMN CDCl₃ ppm
   1,47 (m) - 1,87 (m) : CH₂ centraux ; 2,73 (t), 3,97 : -CH₂-NH₂ ; 7,20 (d) : H'₃ ; 7,50 (d) : H'₅ ; 7,37 (tl) 2H : H₃ H₅ ; 7,24 (it) 1H : H₄ ; 7,77 (m) 2H : H₂et H₆.

### EXEMPLE 2 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(3H-imidazo(4,5-b) pyridin-3-yl) butyl) imino)) érythromycine

On dissout 708,2 mg de 2'-acétate de 11-déoxy 10,11-didéhydro 3-de(2,6-didéoxy 3-C-méthyl 3-O-méthyl alpha-L-ribohexopyranosyl) oxy) 12-O-((1H-imidazol-1-yl) carbonyl) 6-O-méthyl 3-oxo érythromycine préparé comme indiqué à l'exemple 1C de la demande de brevet européen EP 0 596 802 et 958 mg de (3H-imidazo(4,5-b) pyridin-3-butanamine dans 2,82 cm³ d'acétonitrile et 0,28 cm³ d'eau. On porte le mélange réactionnel à 80°C. On laisse revenir à la température ambiante et verse dans une solution de phosphate acide de sodium. On extrait au chlorure de méthylène et lave à l'eau. On rassemble les phases aqueuses et extrait à nouveau. On sèche, filtre, rince et obtient 826 mg de produit. On dissout le produit obtenu dans 16,5 cm³ de méthanol. On maintient la solution réactionnelle sous agitation à la température ambiante pendant 20 heures. On obtient 789 mg de produit recherché brut que l'on purifie par chromatographie en éluant avec le mélange chlorure de méthylène, méthanol, ammoniaque (94-16-0,4). On obtient 327 mg de produit recherché fondant à 200°C.
α_{D} = +13° c = 1 % CHCl₃
RMN CDCl₃ 400 MHz ppm
   0,85 (t) : CH₃-CH₂ ; 1,01 (d)-1,16 (d)-1,25 (d) : les CH₃-CH; 1,30 (d)-1,26 (d), 1,35 et 1,47 : 6 et 12 Me ; ∼ 1,63 et ∼ 1,98 : les CH₂ centraux de la chaîne ; 2,27 (s) : N(CH₃)₂ ; 2,46 (m) : H'₃ ; ∼ 2,59 (m) : H₈ ; 2,61 (s) : 6-OMe ; 3,07 (m) : H₄ ; 3,12 (ql) : H₁₀ ; 3,18 (dd) : H'₂ ; 3,54 (m) : H'₅; 3,57 (s) : H₁₁ ; 3,6 à 3,8 : 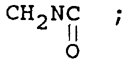 3,85 (q) : H₂ ; 4,24 (d) : H₅ ; 4,29 (d) : H'₁ ; ∼ 4,35 (m) : 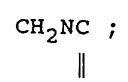 4,93 (dd) : H₁₃ ; 7,21 (dd) : H₆ ; 8,04 (dd) : H₇ aromatiques; 8,11 (s) : H₂ ; 8,38 (dd) : H₅.

### EXEMPLE 3 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(1H-imidazo(4,5-b) pyridin-1-yl) butyl) imino)) érythromycine

On ajoute 708 mg de 2'-acétate de 11-déoxy 10,11-didéhydro 3-de(2,6-didéoxy 3-C-méthyl 3-O-méthyl alpha-L-ribohexopyranosyl) oxy) 12-O-((1H-imidazol-1-yl) carbonyl) 6-O-méthyl 3-oxo érythromycine préparé comme indiqué à l'exemple 1C de la demande de brevet européen EP 0 596 802 dans une solution renfermant 953 mg de (1H-imidazo(4,5-b) pyridine 3-butanamine, 2,82 cm³ d'acétonitrile et 0,28 cm³ d'eau. On porte le mélange réactionnel à 55°C. On maintient à cette température pendant 44 h et ajoute 0,5 cm³ d'acétonitrile. On poursuit le chauffage à 55°C pendant 20 heures. On laisse revenir à la température ambiante et verse sur une solution saturée de phosphate acide de sodium. On extrait la phase aqueuse au chlorure de méthylène et lave à l'eau les phases chlorométhyléniques. On sèche sur sulfate de sodium, filtre et évapore. On obtient 806 mg de produit auquel on ajoute 16,1 cm³ de méthanol. On maintient le mélange réactionnel à la température ambiante pendant 24 heures et évapore à sec. On obtient 656 mg d'un produit que l'on chromatographie sur silice en éluant avec le mélange CH₂Cl₂-MeOH-NH₃ (94-6-0,4).

On obtient le produit recherché brut que l'on purifie par chromatographie sur silice en éluant avec le mélange CHCl₃-MeOH-NH₄OH (94-6-0,4). Après dissolution du résidu dans un mélange acétate d'éthyle-éther isopropylique, filtration et évaporation à sec, on recueille ainsi le produit recherché.
F = 203°C.
α_{D} = 17,6° c = 1 % de CHCl₃.
   0,81 (t) : CH₃-CH₂ ; 1,00 (d)-1,17 (d)-1,25 (d)-1,31 (d)-1,38 (d) : les CH₃-CH ; 1,35 (s)-1,47 (s) : 6 et 12-CH₃ ; 1,68 (m) et 1,93 (m) : CH₂ centraux de la chaîne ; 2,27 (s) : N(CH₃)₂; 2,61 (s) : 6-OCH₃ ; 2,45 (m) : H'₃ ; ∼ 2,60 (m en partie masqué) : H₈ ; 3,07 (m) : H₄ ; ∼ 3,15 (ql) : H₁₀ ; 3,18 (dd): H'₂ ; 3,56 (s) : H₁₁ ; 3,53 (m) : H'₅ ; 3,60 à 3,80 (m) : CO-N-CH₂ ; 3,87 (q) : H₂ ; ∼ 4,25 (m) : CH₂-N-C= ; 4,24 (d) : H₅ ; 4,28 (d) : H'₁ ; 4,91 (dd) : H₁₃ ; 7,21 (dd, J = 5 et 8): H₆ ; 7,80 (dd, J = 8 et 1,5) : H₇ aromatiques ; 8,56 (dd, J = 5 et 1,5) : H₅ ; 8,15 (s) : H₂ + CH₂Cl₂.

### PREPARATION 2 : Préparation des amines utilisées comme produits de départ des exemples 2 et 3 :

### 3H-imidazo(4,5-b)pyridine 3-butanamine et 1H-imidazo(4,5-b) pyridine 1-butanamine

### Stade a :

A une solution de 5,95 g de 4-azabenzimidazole et de 15,5 g de N-4 bromobutylphtalimide dans 30 cm³ de diméthylformamide. On ajoute 10,3 g de carbonate de potassium. Le mélange est agité 20 h à température ambiante. L'insoluble est filtré, rincé au chlorure de méthylène. La phase organique est lavée à l'eau, puis séchée sur sulfate de magnésium et évaporée ; le résidu huileux obtenu est lavé à l'éther de pétrole puis à l'éther isopropylique. On obtient ainsi 16,3 g d'un produit brut qui est purifié par chromatographie sur silice en éluant avec le mélange chlorure de méthylène-acétone pour donner 4,9 g de produit (A), F = 143°C et 3,9 g de produit (B), F = 172°C.

### Stade b1 : 3H-imidazo(4,5-b) pyridine-3-butanamine (produit de départ de l'exemple 2)

On porte au reflux pendant 19 heures, un mélange de 32,86 g de produit (A) préparé comme indiqué au stade précédent, 697 cm³ d'éthanol et 20 cm³ d'hydrazine. On laisse revenir à la température ambiante. On filtre, rince et évapore à sec. On reprend au chlorure de méthylène, filtre, rince et évapore à sec. On obtient 18,87 g de produit recherché.
RMN CDCl₃ - 250 MHz
   1,52 (m)-2,00 (m) : 2 CH₂ centraux ; 1,63 (s large) : 2 H mobiles ; 2,76 (t) : CH₂-CH₂-NH₂ ; 4,33 (t) : 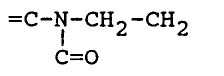 7,24 (dd,J = 8 et 5) : H₆ ; 8,08 (dd,J = 8 et 1,5) : H₇ ; 8,40 (dd,J = 5 et 1,5) : H₅ ; 8,08 (s) : H₂.

### Stade b2 : 1H-imidazo(4,5-b) pyridine 1-butanamine (produit de départ de l'exemple 3)

On porte au reflux pendant 21 heures, un mélange de 32 g de produit (B) de la préparation 3, 640 cm³ d'éthanol et 24,8 cm³ d'hydrazine. On laisse revenir à la température ambiante. On filtre, rince à l'éthanol et évapore sous pression réduite. On reprend au chlorure de méthylène, filtre, rince et évapore à sec. On obtient 19,5 g de produit recherché.
RMN CDCl₃
   1,45 (m)-1,96 (m) : 2 CH₂ centraux ; 2,74 (t) : CH₂-NH₂ ; - 1,45 (m) : mobile ; 4,23 (t) : 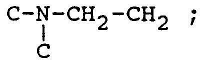 7,24 (dd, J = 8 et 5) : H₆ ; 7,75 (dd, J = 8 et 1,5) : H₇ ; 8,58 (dd, J = 5 et 1,5) : H₅ ; 8,13 (s) : H₂ + EtOH.

En opérant comme précédemment, en utilisant les amines appropriées, on a préparé les produits suivants :

### EXEMPLE 4 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(thieno(2,3-b) pyridin-4-yl) butyl) imino)) érythromycine

F = 176 - 178°C.
α_{D} = +17° c = 0,9 % dans CHCl₃

### EXEMPLE 5 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(3-phényl 1H-1,2,4-triazol-1-yl) butyl) imino)) érythromycine

F = 208 - 210°C.
α_{D} = +17° c = 1 % dans CHCl₃

### EXEMPLE 6 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(1-méthyl-1H-imidazo(4,5-c)pyridin-2-yl) butyl) imino)) érythromycine

α_{D} = + 19° c = 1 % CHCl₃

### EXEMPLE 7 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(3-méthyl-3H-imidazo(4,5-c)pyridin-2-yl) butyl) imino)) érythromycine

α_{D} = + 16° CHCl₃ = 1 %

### EXEMPLE 8 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(5-phényl-1H-tétrazol-1-yl) butyl) imino)) érythromycine

F = 132 - 134°C.
α_{D} = +25° c = 1 % CHCl₃

### EXEMPLE 9 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(2-benzothiazolyl) butyl) imino)) érythromycine

F = 179 - 181°C.
α_{D} = +18° c = 1 % CHCl₃

### EXEMPLE 10 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(2-(3-pyridinyl) 4-thiazolyl) butyl) imino)) érythromycine

F = 150 - 152°C.
α_{D} = +17° c = 0,9 % CHCl₃

### EXEMPLE 11 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(2-(3-pyridinyl) 5-thiazolyl) butyl) imino)) érythromycine

F = 155 - 159°C.
α_{D} = +12° c = 1 % CHCl₃

### EXEMPLE 12 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(9H-purin-9-yl) butyl) imino)) érythromycine

### EXEMPLE 13 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(1H-imidazo(4,5-c) pyridin-1-yl) butyl) imino)) érythromycine

rf = 0,42 CHCl₃ + 8 % de MeOH à 8 % de NH₄OH

### EXEMPLE 14 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((5-(1H-benzimidazol-1-yl) pentyl) imino)) érythromycine

Préparé à partir du 2-(4-bromopentyl) 1H-iso-indole 1,3(2H)-dione.

### EXEMPLE 15 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((5-chloro-1H-benzimidazol-1-yl) butyl) imino)) érythromycine

F = 145-148°C.

### EXEMPLE 16 : 11,12-didéoxy 3-de((2,6-didéoxy-3-C-méthyl-3-O-méthyl-alpha-L-ribohexopyranosyl) oxy) 6-O-méthyl 12,11-(oxycarbonyl ((4-(1H-indol-1-yl) butyl) imino)) érythromycine

### EXEMPLE 17 : 11,12-didéoxy 3-de((2,6-didéoxy-3-C-méthyl-3-O-méthyl-alpha-L-riboxopyranosyl) oxy) 6-O-méthyl 3-oxo 12,11-(oxycarbonyl ((4-(1-méthyl-1H-indol-4-yl) butyl) imino)) érythromycine

α_{D} = 20 %, c = 1 % CHCl₃

### EXEMPLE 18 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(2-phényl-4-quinoleinyl) butyl) imino)) érythromycine

F = 195-197°C.

### EXEMPLE 19 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(1H-benzotriazol-1-yl) butyl) imino)) érythromycine

F = 200-202°C.

### EXEMPLE 20 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(2H-benzotriazol-2-yl) butyl) imino)) érythromycine

F = 164-166°C.

### EXEMPLE 21 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(5,6-diméthyl 1H-benzimidazol-1-yl) butyl) imino)) érythromycine

F = 174-176°C.

### EXEMPLE 22 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(3-quinoléinyl) butyl) imino)) érythromycine

F = 195-197°C.

### EXEMPLE 23 : 11,12-didéoxy 3-de((2,6-didéoxy-3-C-méthyl-3-O-méthyl-alpha-L-ribohexopyranosyl) oxy) 6-O-methyl 3-oxo 12,11-(oxycarbonyl ((4-(2-quinoléinyl) butyl) imino)) érythromycine

F = 179-181°C.

### EXEMPLE 24 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(2-méthyl 1H-benzimidazol-1-yl) butyl) imino)) érythromycine

F = 128-132°C.

### EXEMPLE 25 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(6-chloro 1H-benzimidazol-1-yl) butyl) imino)) érythromycine

F = 192-194°C.

### EXEMPLE 26 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(1-méthyl 1H-benzimidazol-2-yl) butyl) imino)) érythromycine

### EXEMPLE 27 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(5H-imidazo(4,5-c)pyridin-5-yl) imino)) érythromycine

α_{D} = 12,2 c = 1 % CHCl₃

### EXEMPLE 28 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-(4-chlorophényl) 1H-imidazol-1-yl) butyl) imino)) érythromycine

On chauffe 7 heures à 75°C, 1 g de 2'-acétate de 11-déoxy 10,11-didéhydro 3-de(2,6-didéoxy 3-C-méthyl 3-O-méthyl alpha-L-ribohexopyranosyl) oxy) 12-O-((1H-imidazol-1-yl) carbonyl) 6-O-méthyl 3-oxo érythromycine préparée comme indiqué à l'exemple 1C de la demande de brevet européen EP 0 596 802 dans 4 cm³ d'acétonitrile à 10% d'eau avec 1,4 g de 4-(4-(4-chlorophényl) 1H-imidazol) butanamine. On laisse revenir à température ambiante, dilue à l'eau, extrait à l'acétate d'éthyle, sèche, évapore le solvant et obtient 2,3 g de produit acétylé en 2'. On ajoute 60 ml de méthanol et maintient 16 heures sous agitation, évapore le solvant, chromatographie le résidu sur silice (éluant : CH₂Cl₂-MeCH-NH₄OH 95-5-0,4), concentre et cristallise le résidu dans l'éther. On sèche le produit cristallisé sous pression réduite à 80°C et récupère 381 mg de produit attendu. F = 192-194°C.
RMN CDCl₃ ppm
   0,83 (t) : CH₃-CH₂ ; 1,00 (d)-1,16 (d)-1,24 (d)-1,30 (d)-1,38 (d) : les CH₃-CH ; 1,33 (s)-1,47 (s) : 6 et 12 Me ; 2,26 (s): N(Me)₂ ; 2,44 (m) : H'₃ ; 2,61 (s) : 6-OMe ; 2,60 (m) : H₈ ; 3,00 à 3,21 : H₄, H₁₀ et H'₂ ; 3,55 (m) : H'₅ ; 3,56 (s) : H₁₁ ; 3,60 à 3,80 2H-3,99 (t) 2H : 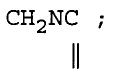 3,87 (q) : H₂ ; 4,23 (d) : H₅ ; 4,28 (d) : H'₁ ; 4,93 (dd) : H₁₃ ; 7,26 (d) : H₅ imidazole ; 7,50 (d) : H₂ imidazole ; 7,32-7,70 : aromatiques ; 3,51 : OH.

### Préparation de la 4-(4-chlorophényl) 1H-imidazole 1-butanamine utilisé au départ de l'exemple 28.

### Stade A : 4-(4-chlorophényl) 1H-imidazole.

On porte au reflux pendant 1 heure 23,34 g de ω-bromo 4-chloro acétophénone dans 150 ml de formamide ; on laisse refroidir, alcalinise avec une solution de soude, extrait au dichlorométhane, lave à l'eau, sèche, évapore le solvant, chromatographie le résidu sur silice (éluant : CH₂Cl₂-MeOH-NH₄OH 8-2-0,04) et obtient 13,4 g de produit attendu. F = 146-148°C.

### Stade B : 2-(4-(4-(4-chlorophényl) 1H-imidazol 1-yl) 1H-iso indol-1,3(2H)-dione.

On opère comme au stade A de la préparation de l'exemple 1 en utilisant 12,2 g du produit obtenu au stade A, 4,96 g d'hydrure de sodium et 23,83 g de N-4-bromobutyl phtalimide. On obtient 9,7 g de produit attendu.

### Stade C : 4-(4-chlorophényl) 1H-imidazol 1-butanamine.

On opère comme au stade B de la préparation de l'exemple 1 en utilisant 14,2 g de produit obtenu comme au stade B ci-dessus et 3,6 ml d'hydrate d'hydrazine dans 200 ml d'éthanol. On obtient 12 g de produit brut que l'on chromatographie sur silice (éluant : CH₂Cl₂-MeOH-NH₄OH 8-2-0,04) et obtient le produit utilisé tel quel pour la synthèse.
RMN (CDCl₃) ppm
   1,22 (sl) : 2H mobiles ; 1,47 (m)-1,88 (m) : 2 CH₂ centraux ; 2,74 (m) : CH₂-CH₂-N ; 3,98 (m) : 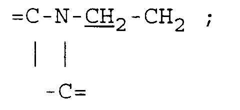 7,19 (d, J=1,5)-7,50 (d, J=1,5) : H₂ et H₅ ; 7,33 et 7,70 : aromatiques.

### EXEMPLE 29 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-(2-méthoxyphényl) 1H-imidazol-1-yl) butyl) imino)) érythromycine

On chauffe à 80°C pendant 8 heures 706 mg de composé de départ de l'exemple 28 (obtenu comme indiqué à l'exemple 1C de la demande de brevet européen EP 0 596 802) dans 3 ml d'acétonitrile et 908 mg de 4-(4-(2-méthoxyphényl) 1H-imidazol-1-yl) butanamine. On laisse revenir à température ambiante, verse sur une solution d'hydrogénophosphate de sodium (0,5M), extrait à l'acétate d'éthyle, lave à l'eau, sèche, évapore le solvant, obtient 1,6 g de produit acétylé en 2'. On y ajoute 50 ml de méthanol, agite pendant 16 heures, évapore le solvant, chromatographie le résidu sur silice (éluant : AcOEt-TEA à 4%) et cristallise dans l'éther. On obtient 194 mg de produit attendu. F = 143-145°C.
RMN CDCl₃ ppm
   0,85 (t) : CH₃-CH₂ ; 1,01 (d)-1,16 (d)-1,24 (d)-1,30 (d)-1,37 (d) : les CH₃-CH ; 1,34 (s)-1,47 (s) : 6 et 12 Me ; 2,26 (s): N(Me)₂ ; 2,44 (m) : H'₃ ; 2,60 (m) : H₈ ; 2,64 (s) : 6-OMe ; 3,08 (m) : H₄ ; 3,12 (ql) : H₁₀ ; 3,17 (dd) : H'₂ ; 3,54 (m) : H'₅ ; 3,57 (s) : H₁₁ ; 3,66 (m)-3,74 (m) : 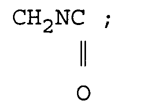 3,85 (q) : H₂ ; 3,95 (s) : Φ-OMe ; 3,99 (ql) : CH₂-N-C= ; 4,24 (d) : H₅ ; 4,27 (d) : H'₁ ; 4,93 (dd) : H₁₃ ; 6,97 (dl) : H₆ ; 7,51 (s) : les H imidazole ; 7,02 : H₆ phényl ; 7,19 (ddd) H₄ et H₅ phényl ; 8,19 (dd) : H₂.

### Préparation de la 4-(2-méthoxyphényl) 1H-imidazol-1-butanamine utilisée au départ de l'exemple 29.

### Stade A : 4-(2-méthoxyphényl) 1H-imidazole.

On chauffe au reflux 9,36 g de 2-bromo 2'-méthoxyacétophénone dans 50 ml de formamide, laisse revenir à température ambiante, lave avec une solution d'acide chlorhydrique 2N, filtre, alcalinise jusqu'à pH 8-9 à l'aide de soude 2N, extrait au dichlorométhane, lave à l'eau, sèche, évapore le solvant, chromatographie le résidu sur silice (éluant : CH₂Cl₂-MeOH-NH₄OH 95-5-0,4) et obtient 6,15 g de produit attendu.

### Stade B : 2-(4-(4-(2-méthoxyphényl) 1H-imidazol 1-yl) butyl 1H-iso indol-1,3(2H-dione.

On opère comme au stade A de la préparation de l'exemple 1 en utilisant 6 g du produit obtenu au stade A, 1,99 g d'hydrure de sodium et 9,93 g de N-4-bromobutyl phtalimide. On obtient 6,15 g de produit attendu.

### Stade C : 4-(2-méthoxyphényl) 1H-imidazol 1-butanamine (fumarate).

On opère comme au stade B de la préparation de l'exemple 1 en utilisant 5,65 g de produit obtenu comme au stade B ci-dessus et 1,45 ml d'hydrate d'hydrazine dans 75 ml d'éthanol. On obtient 3,8 g de produit brut que l'on dissout dans 4 ml de tétrahydrofuranne puis ajoute 1,87 g d'acide fumarique en solution dans 20 ml de méthanol. On ajoute 10 ml d'éther, essore les cristaux formés, les sèche à 80°C sous pression réduite et récupère 3,77 g de fumarate du produit attendu.
F = 160-162°C.
RMN (CDCl₃) ppm
   1,48 (m) 2H-1,87 (m) 2H : les CH₂ centraux ; 3,46 : NH₂ ; 2,73 (t) : CH₂N ; 3,94 (s) : Φ-OMe ; 3,97 (t) : 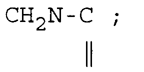 6,94 (dd) : H₆ ; 7,04 (dt)-7,21 (ddd) : H₅ et H₄ ; 7,51 : H'₂ et H'₅ ; 8,19 (dd): H₂.

### EXEMPLE 30 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-(4-fluorophényl) 1H-imidazol-1-yl) butyl) imino)) érythromycine

On chauffe à 60°C pendant 4 heures et demie 2,11 g de composé de départ de l'exemple 28 (obtenu comme indiqué à l'exemple 1C de la demande de brevet européen EP 0 596 802) dans 9 ml d'acétonitrile et 2,8 g de 4-(4-(4-fluorophényl) 1H-imidazol-1-yl) butanamine. On laisse revenir à température ambiante, verse dans l'eau, extrait à l'acétate d'éthyle, lave à l'eau, sèche, évapore le solvant, obtient 5,2 g de produit acétylé en 2'. On y ajoute 20 ml de méthanol, agite pendant 3 heures et demie, évapore le solvant, chromatographie le résidu sur silice (éluant : CH₂Cl₂-MeOH-NH₄OH 95-5-0,3) et cristallise dans l'éther. On obtient 1,34 g de produit attendu. F = 190-192°C.
RMN CDCl₃ ppm
   1,33 (s)-1,47 (s) : 6 et 12 Me ; 2,27 (s) : N(Me)₂ ; 2,61 (s) : 6-OMe ; 3,0 à 3,18 : H₄ et H₁₀ ; 3,56 (s) : H₁₁ ; 3,59 à 3,81 : 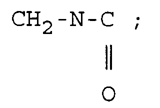 3,98 (t) : 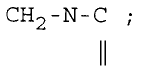 ∼ 7,05 - ∼7,73 : fluorophényl ; 7,21 (d) : H₅ imidazole ; 7,49 (d) : H₂ imidazole.

### Préparation de 4-(4-fluorophényl) 1H-imidazol-1-butanamine utilisée au départ de l'exemple 30.

### Stade A : 4-(4-fluorophényl) 1H-imidazole.

On chauffe 2 heures au reflux 10,85 g de bromure de 4-fluorophénacyle dans 60 ml de formamide, laisse revenir à température ambiante, acidifie jusqu'à pH 2 à l'aide d'acide chlorhydrique N, filtre, neutralise par addition d'ammoniaque, extrait au dichlorométhane, lave à l'eau, sèche, évapore le solvant, chromatographie le résidu sur silice (éluant : CH₂Cl₂-MeOH-NH₄OH 95-5-0,4) et obtient 5,8 g de produit attendu. F = 130-132°C.

### Stade B : 2-(4-(4-(4-fluorophényl) 1H-imidazol 1-yl) butyl 1H-iso indol-1,3(2H-dione.

On opère comme au stade A de la préparation de l'exemple 1 en utilisant 10 g du produit obtenu au stade A, 1,95 g d'hydrure de sodium et 11,80 g de N-4-bromobutyl phtalamide. On obtient 7,53 g de produit attendu. F = 138-140°C.

### Stade C : 4-(4-fluorophényl) 1H-imidazol 1-butanamine.

On opère comme au stade B de la préparation de l'exemple 1 en utilisant 3,64 g de produit obtenu comme au stade B ci-dessus et 1 ml d'hydrate d'hydrazine dans 80 ml d'éthanol. On obtient 2,4 g de produit brut que l'on chromatographie sur sicile (éluant : CH₂Cl₂-MeOH-NH₄OH 8-2-0,03) et obtient le produit utilisé tel quel pour la synthèse.
RMN (CDCl₃) ppm
   1,48 (m)-1,81 (m) : les CH₂ centraux ; 2,74 (t) : N-CH₃ ; 3,98 (t) : >N-CH₂-CH₂ ; 7,06 (t) : >CH-F ; 7,22 (m) : 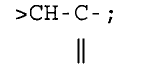 7,49 (s) : H₂ imidazole ; 7,15 (s) : H₅ imidazole.

### EXEMPLE 31 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(7-méthoxy (4-quinoléinyl) butyl) imino)) érythromycine

On chauffe à 50°C pendant 53 heures 706 mg de composé de départ de l'exemple 29 (obtenu comme indiqué à l'exemple 1C de la demande de brevet européen EP 0 596 802) dans 4 ml d'acétonitrile et 1,43 g de 7-méthoxy-4-quinoléinebutanamine. On laisse revenir à température ambiante, verse sur une solution d'hydrogénophosphate de sodium (0,5M), extrait au dichlorométhane, lave à l'eau, sèche, évapore le solvant, obtient 1,09 g de produit acétylé en 2'. On y ajoute 10 ml de méthanol, agite pendant 16 heures, évapore le solvant, chromatographie le résidu sur silice (éluant : CH₂Cl₂-MeOH 95-5) et cristallise dans l'éther. On obtient 295 mg de produit attendu. F ≈ 110°C.
RMN CDCl₃ ppm
   3,06 (m) : -(CH₂)₂-CH< ; 3,70 (m) : -N-CH₂- ; 3,95 (s) : -OCH₃ ; 7,12 (d)-7,19 (dd)-7,42 (d)-7,94 (d)-8,70 (d) : pyridine.

### Préparation de la 7-méthoxy-4-quinoléinebutanamine utilisée au départ de l'exemple 31.

### Stade A : Sel de triphényl phosphonium du N-(3-bromopropyl) phtalimide.

On chauffe au reflux pendant 44 heures 13,4 g de N-bromopropylphtalimide et 13,15 g de triphénylphosphine en suspension dans 75 ml de xylène. On laisse revenir à température ambiante, essore le précipité, le lave à l'éther éthylique et sèche sous pression réduite à 60°C. On récupère 24,88 g de produit attendu. F = 220-222°C.

### Stade B : (Z)-(2-[4-(7-méthoxy-4-quinoléinyl)3-butényl]1H-isoindole-1,3(2H)-dione.

On ajoute 4 g de 7-méthoxy 4-quinoléinylcarboxaldéhyde dans une suspension de 12,47 g de sel de triphénylphosphonium de 3-bromopropyl phtalimide dans 200 ml de tétrahydrofuranne. On refroidit à -50°C, ajoute 2,72 g de terbutylate de potassium, laisse remonter lentement la température à -6°C, filtre, concentre le filtrat, reprend le résidu dans l'acétate d'éthyle, lave à l'eau, sèche, évapore le solvant et récupère 9,26 g de produit brut que l'on chromatographie sur silice (éluant : CHCl₃-AcOEt 80-20 puis 70-30). On récupère 3,575 g de produit attendu.

### Stade C : 2-(4-(7-méthoxy 4-quinoléinyl) butyl) 1H-isoindol 1,3(2H)-dione.

On dissout 3,50 g de produit obtenu au stade B dans 50 ml de méthanol, ajoute 0,36 g de palladium sur charbon actif et hydrogène 3 heures sous 600 mbars. On filtre, évapore le solvant et recueille 3,48 g de produit attendu.

### Stade D : 7-méthoxy quinoléin-4-butanamine.

On dissout à chaud 3,46 g de produit obtenu au stade C dans 70 ml d'éthanol, ajoute 1,86 ml d'hydrate d'hydrazine, porte au reflux pendant 17 heures, élimine par filtration le précipité, évapore le solvant, reprend le résidu par 70 ml de dichlorométhane, filtre, évapore le solvant et recueille 2,19 g de produit attendu.
RMN (CDCl₃) ppm
   1,6 (m)-1,79 (m) : CH₂ centraux ; 2,75 (t) : >-CH₂-(CH₂)₃ ; 3,05 (t) : CH₂-NH₂ ; 3,95 (s) : O-CH₃ ; 7,10 (d, J=4,5)-7,21 (dd)-7,92 (d)-8,71 (d, J=4,5) : quinoléine.

### EXEMPLE 32 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(2-(2-pyridinyl) 4-thiazolyl) butyl) imino)) érythromycine

On chauffe à 60°C pendant 5 heures 705 mg de composé de départ de l'exemple 28 (obtenu comme indiqué à l'exemple 1C de la demande de brevet européen EP 0 596 802) dans 3 ml d'acétonitrile et 0,705 g de 4-(2-(2-pyridinyl 4-thiazolyl) butanamine. On laisse revenir à température ambiante, verse dans l'eau, extrait à l'acétate d'éthyle, lave à l'eau, sèche, évapore le solvant, obtient 1,8 g de produit acétylé en 2'. On y ajoute 15 ml de méthanol, chauffe au reflux pendant 2 heures, évapore le solvant, chromatographie le résidu sur silice (éluant : CH₂Cl₂-MeOH-NH₄OH 95-5-0,3 puis AcOEt-TEA 9-1) et cristallise dans l'éther. On obtient 194 mg de produit attendu. F = 157-159°C.
RMN (CDCl₃) ppm
   1,33 et 1,47 : 6 et 12 Me ; 2,26 (s) : N(CH₃)₂ ; 2,86 (t) : 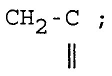 3,12(ql) : H₁₀ ; 3,60 (s) : H₁₁ ; 3,66 (m) : 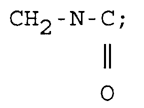 7,03 (s) : H₅ thiazole ; 7,27 (ddd) : H₅ pyridyne ; 7,77 (dt): H₄ pyridyne ; 8,18 (dd) H₃ pyridyne ; 8,53 (ddd) : H₆ pyridyne.

### Préparation de la 2-(2-pyridinyl)-4-thiazolebutanamine utilisée au départ de l'exemple 32.

### Stade A : 2-aminocarbonyl pyridine.

On ajoute goutte à goutte 50 ml d'une solution diazométhane (0,4 M/l) dans une solution comprenant 2 g d'acide picolinique, 20 ml de dichlorométhane et 5 ml de méthanol. Après 30 minutes d'agitation à température ambiante, on évapore le solvant sous pression réduite, chromatographie le résidu sur silice (éther de pétrole (60-80)-AcOEt 5-5) et récupère 1,48 g d'ester méthylique. On chauffe à 50°C pendant 4 heures 1,42 g d'ester dans 5 ml d'ammoniaque, laisse revenir à température ambiante, extrait à l'éther, lave à l'eau, sèche, évapore le solvant et récupère 1,05 g de produit attendu. F = 105°C.

### Stade B : 2-pyridine carbothioamide.

On ajoute lentement 43 g de pentasulfure de phosphore à 46,8 g de l'amide obtenue au stade A dans 700 ml de tétrahydrofuranne. On agite 4 heures à température ambiante, verse dans l'eau, extrait à l'éther, sèche et évapore le solvant sous pression réduite. Après chromatographie sur silice (éluant : CH₂Cl₂-AcOEt 8-2), on recueille 10 g de produit attendu. F = 137°C.

### Stade C : 2-(2-pyridinyl) 4-thiazole carboxylate d'éthyle.

On ajoute goutte à goutte 16,3 ml de bromopyruvate d'éthyle à 15,9 g du produit préparé comme au stade B dans 250 ml d'éthanol et chauffe 5 heures au reflux. On évapore le solvant sous pression réduite, chromatographie le résidu sur silice (éluant : hexane-AcOEt 1-1) et obtient 10,2 g de produit attendu. F = 69,1°C.

### Stade D : 2-(2-pyridinyl) 4-thiazole méthanol.

On ajoute lentement 40 ml de méthanol dans un mélange comprenant 9,3 g d'ester préparé au stade C et 4,1 g de borohydrure de sodium dans 100 ml de tétrahydrfuranne et chauffe 2 heures au reflux. On laisse revenir à température ambiante, verse dans l'eau, neutralise à l'aide d'acide chlorhydrique N, extrait au dichlorométhane, sèche la phase organique et évapore le solvant sous pression réduite, chromatographie le résidu sur silice (éluant : AcOEt-CH₂Cl₂1-1) et obtient 5,8 g de produit attendu. F = 100°C.

### Stade E : 2-(2-pyridinyl) 4-thiazole carboxaldéhyde.

On chauffe 2 heures au reflux 5,8 g du produit obtenu au stade D dans 60 ml de toluène en présence de 13 g d'oxyde de manganèse, filtre et évapore le solvant sous pression réduite. On obtient 5 g de produit attendu. F = 131°C.

### Stade F : (Z) 2-(4-(2-(2-pyridinyl) 4-thiazolyl) 3-butényl) 1H-isoindole 1,3(2H)-dione.

On opère comme au stade A de la préparation 31 en utilisant 5,70 g de l'aldéhyde préparé comme au stade E ci-dessus et 15,9 g de sel de triphénylphosphonium de 3-bromopropyl phosphonium et 3,70 g de terbutylate de potassium. On obtient 8,73 g de produit attendu. F = 139-141°C.

### Stade G : (2-(4-(2-(2-pyridinyl) 4-thiazolyl) butyl) 1H-isoindol 1,3(2H)-dione.

On opère comme au stade B de la préparation 31 en utilisant au départ 7,22 g de produit préparé au stade F ci-dessus, et 1,5 g de palladium sur charbon actif en hydrogénant 2 heures sous 1800 mbars. On obtient 6,33 g de produit attendu. F = 119-121°C.

### Stade H : 2-(2-pyridinyl)-4-thiazolebutanamine.

On opère comme au stade C de la préparation 31 en utilisant 5,45 g du produit obtenu au stade G ci-dessus et 1,6 ml d'hydrate d'hydrazine et en chauffant 6 heures au reflux. On évapore le solvant, reprend par de l'acétate d'éthyle, lave à l'eau, sèche, évapore le solvant, chromatographie le résidu sur silice (éluant : CH₂Cl₂-MeOH-NH₄OH 9-1-0,03) et obtient 1,65 g de produit attendu.
RMN (CDCl₃) ppm
   1,50 (m)-1,82 (m) : les CH₂ centraux ; 2,76 (t)-2,85 (t) : CH₂-C= et CH₂-NH₂ ; 7,85 (s) : H₅ thiazole ; 7,31 (m) : H'₅ ; 7,78 (dt) : H'₄ ; 8,18 (dt) : H'₃ ; 8,61 (ddd) : H'₆ ; 1,40 (s) : NH₂.

### EXEMPLE 33 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-(3-pyridinyl) 1H-imidazol-1-yl) butyl) imino)) érythromycine

On chauffe à 70°C pendant 20 heures 1 g de composé de départ de l'exemple 28 (obtenu comme indiqué à l'exemple 1C de la demande de brevet européen EP 0 596 802) dans 4 ml d'acétonitrile et 936 mg de 4-(3-pyridinyl)-1H-imidazole-1-butanamine. On laisse revenir à température ambiante, verse dans l'eau, extrait à l'acétate d'éthyle, lave à l'eau, sèche, évapore le solvant, obtient 1,34 g de produit acétylé en 2'. On y ajoute 40 ml de méthanol, agite pendant 2 heures, évapore le solvant, chromatographie le résidu sur silice (éluant : CH₂Cl₂-MeOH-NH₄OH 95-5-0,4) et cristallise dans l'éther. On obtient 310 mg de produit attendu. F = 187-188°C.
RMN (CDCl₃) ppm
   0,83 (t) : CH₃-CH₂ ; 1,01 (d)-1,17 (d)-1,25 (d)-1,31 (d)-1,38 (d) : les CH₃-CH ; 1,34 (s)-1,47 (s) : 6 et 12 Me ; 2,27 (s): N(Me)₂ ; 2,45 (m) : H'₃ ; 2,62 (s) : 6-OMe ; 2,60 (m) : H₈ ; 2,85 à 3,25 : H₄ et H₁₀, H'₂ ; 3,52 (m) : H'₅ ; 3,56 (s) : H₁₁ ; 3,60 à 3,85 (m) : 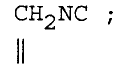 4,23 (d) : H₅ ; 4,27 (d) : H'₁ ; 4,93 (dd) : H₁₃ ; 7,29 (ddd) : H₅ pyridine ; 8,08 (dt) : H₄ pyridinye ; 8,45 (dd) : H₆ pyridine ; 8,97 (dd) : H₂ pyridine ; 7,35 (d) et 7,53 (d) : H₂ et H₅ imidazole.

### Préparation de la 4-(3-pyridinyl) 1H-imidazole-1-butanamine utilisée au départ de l'exemple 33.

### Stade A : 2-(4-(3-pyridinyl) 1H-imidazol 1-yl) butyl 1H-iso indol-1,3(2H)-dione.

On opère comme au stade A de la préparation de l'exemple 1 en utilisant 290 mg de 3-pyridinyl 1H-imidazole préparé comme indiqué dans J. Chem. Soc. 753-5 (1938), 115 mg d'hydrure de sodium et 633 mg de N-4-bromobutyl phtalimide. On obtient 277 mg de produit attendu. F = 150-152°C.

### Stade B : 4-(3-pyridinyl) 1H-imidazol 1-butanamine.

On opère comme au stade B de la préparation de l'exemple 1 en utilisant 1,66 g de produit obtenu comme au stade A ci-dessus et 0,46 ml d'hydrate d'hydrazine dans 30 ml d'éthanol. On obtient 936 mg de produit utilisé tel quel pour la synthèse.
RMN (CDCl₃) ppm
   1,49 (m)-1,89 (m) : les CH₂ centraux ; 2,75 (t) : CH₂-CH₂-N ; 4,01 (t) : 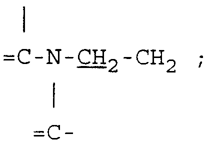 7,29 (d, J=1)-7,55 (d, J=1) : H₂ et H₅ ; 7,30 (en partie masqué) : H'₅ ; 8,09 (dt, J=8 et 2) : H'₄ ; 8,47 (dd, J=5 et 2) : H'₆ ; 8,96 (d, J=2) : H'₂ ; 1,49 (sl) : ≈ 2H mobiles.

### EXEMPLE 34 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(3-(3-pyridinyl) 1H-1,2,4-triazol-1-yl) butyl) imino)) érythromycine

On chauffe à 75°C pendant 8 heures 1 g de composé de départ de l'exemple 28 (obtenu comme indiqué à l'exemple 1C de la demande de brevet européen EP 0 596 802) dans 4 ml d'acétonitrile et 1,21 g de 4-(3-(3-pyridinyl 1H-1,2,4-triazol-1-yl) butanamine. On laisse revenir à température ambiante, verse dans l'eau, extrait à l'acétate d'éthyle, lave à l'eau, sèche, évapore le solvant, obtient 2 g de produit acétylé en 2'. On y ajoute 40 ml de méthanol, agite pendant 16 heures, évapore le solvant, chromatographie le résidu sur silice (éluant : CH₂Cl₂-MeOH-NH₄OH 90-10-0,04) et cristallise dans l'éther. On obtient 292 mg de produit attendu. F = 190-192°C.
RMN (CDCl₃) ppm
   0,84 (t) : CH₃-CH₂ ; 1,01 (d) : OMe ; 1,16 (d) : 8Me ; 1,25 (d) : 5Me ; 1,30 (d) : 4Me ; 1,34 (d) : 2Me ; 1,33 (s)et 1,47 (s) : 6 et 12 Me ; 1,67 (m)-1,99 (m) : les CH₂ centraux ; 2,26 (s): N(Me)₂ ; 2,44 (m) : H'₃ ; 2,58 (m) : H₈ ; 2,61 (s): 6-OMe ; 3,06 (m) : H₄ ; 3,12 (q) : H₁₀ ; 3,17 (dd) : H'₂ ; 3,52 (m) : H'₅ ; 3,56 (s) : H₁₁ ; 3,64 à 3,75 (-) : 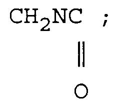 3,85 (q) : H₂ ; ∼ 4,25 : H'₁, H₅ et 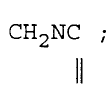 4,91 (dd) : H₁₃ ; 8,15 (s) : H triazole ; 7,35 (dd) : H₅ pyridine ; 8,34 (dt) : H₄ pyridine ; 8,62 (dd) : H₆ pyridine; 9,31 (dl) : H₂ pyridine.

### Préparation de la 3-(3-pyridinyl) 1H-1,2,4-triazol-1-butanamine utilisée au départ de l'exemple 34.

### Stade A : 2-(4-(3-(3-pyridinyl) 1H-1,2,4-triazol-1-yl) butyl 1H-isoindol-1,3(2H)-dione.

On opère comme au stade A de la préparation de l'exemple 1 en utilisant 2,1 g de 3-pyridinyl 1H-1,2,4-triazole préparé comme indiqué dans J. Org. Chem. (44) n° 33, 4160-4164 (1979), 1,02 g d'hydrure de sodium et 4,13 g de N-4-bromobutyl phtalimide. On obtient 2,4 g de produit attendu.
F = 150-152°C.

### Stade B : 3-(3-pyridinyl) 1H-1,2,4-triazol-1-butanamine (fumarate).

On opère comme au stade B de la préparation de l'exemple 1 en utilisant 3,46 g de produit obtenu comme au stade A ci-dessus et 1 ml d'hydrate d'hydrazine dans 50 ml d'éthanol. On obtient 2,1 g de produit brut que l'on transforme en fumarate comme indiqué à la préparation 30 et obtient 1,13 g de fumarate du produit attendu. F ≈ 190-192°C.
RMN (CDCl₃) ppm
   1,50 (m)-2,01 (m) : les CH₂ centraux ; 2,76 (t) : NH₂-CH₂- ; 4,24 : =N-N-CH₂ ; 7,37 (ddd) : H₅ ; 8,35 (dt) : H₄ ; 8,63 (dd) : H₆ ; 9,32 (dd) : H₂ ; 8,12 (s) : =CH triazole.

En opérant comme précédemment en utilisant les amines appropriées, on a préparé les produits suivants :

### EXEMPLE 35 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(3-quinoléinyl) butyl) imino)) érythromycine

F = 190-192°C.

### EXEMPLE 36 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-(4-méthoxyphényl) 1H-imidazol-1-yl) butyl) imino)) érythromycine

F = 152-154°C.

### EXEMPLE 37 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(2-phényl 4-thiazolyl) butyl) imino)) érythromycine

F = 141-143°C.

### EXEMPLE 38 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-(3-méthoxyphényl) 1H-imidazol-1-yl) butyl) imino)) érythromycine

F = 144-146°C.

### EXEMPLE 39 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4,5-diphényl) 1H-imidazol-1-yl) butyl) imino)) érythromycine

F = 180-182°C.

### EXEMPLE 40 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-quinazolinyl) butyl) imino)) érythromycine

F = 212-214°C.

### EXEMPLE 41 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(2-(4-pyridinyl) 4-thiazolyl) butyl) imino)) érythromycine

F = 192-194°C.

### EXEMPLE 42 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(1,2,3,6-tétrahydro-1,3-diméthyl 2,6-dioxo 7H-purin-7-yl) butyl) imino)) érythromycine

F = 251-253°C.

### EXEMPLE 43 : 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-(4-trifluorométhoxy) phényl) 1H-imidazol-4-yl) butyl) imino)) érythromycine

F = 168-170°C.
Les amines utilisées comme produits de départ sont préparées selon les méthodes suivantes :
**A** - Lorsque la chaîne est attachée sur un carbone par exemple 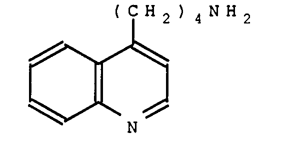 on peut partir des aldéhydes correspondants : 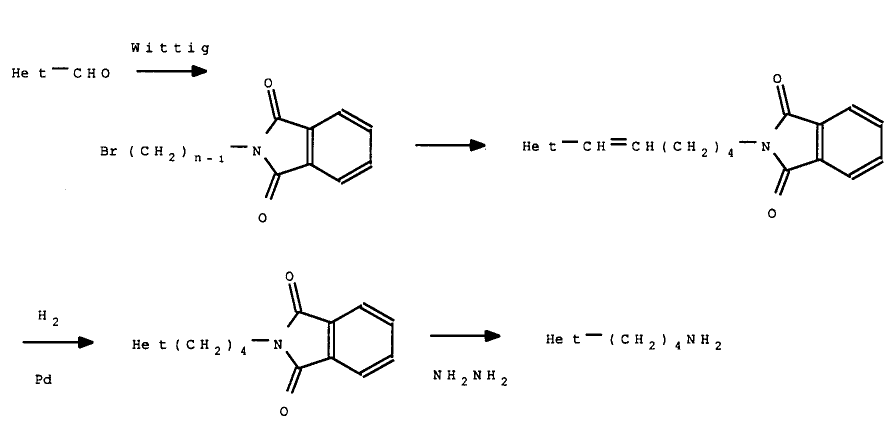
   Les amines utilisées pour la préparation des produits des exemples 4, 31, 10, 11, 17, 18, 22 et 24 ont été préparées de cette façon.
**B** - Lorsque la chaîne est attachée à un azote, on peut préparer les amines de la façon suivante : 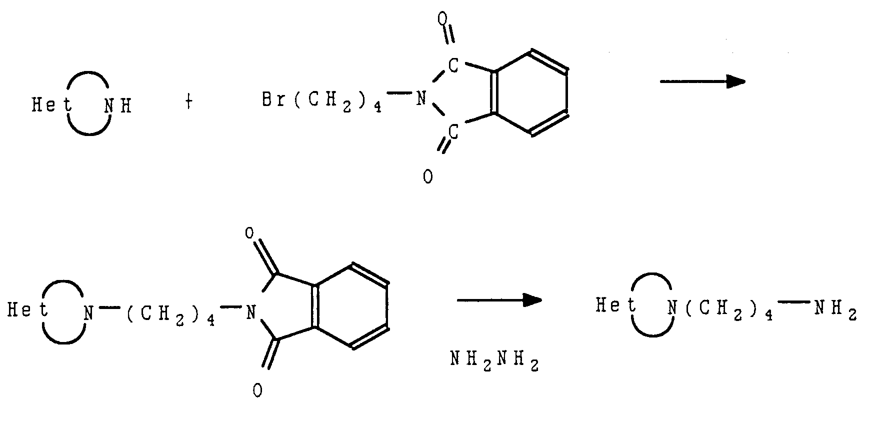
   Les amines utilisées pour la préparation des produits des exemples 1, 2, 3, 5, 9, 13, 14, 15, 16, 17, 20, 21, 22, 25, 26 et 28 ont été préparées de cette façon.
**C** - Certaines amines sont préparées de façon particulière : on construit l'hétérocycle et l'on introduit la chaîne en même temps (exemples 6, 7, 10 et 27).

### EXEMPLES DE COMPOSITIONS PHARMACEUTIQUES

On a préparé des composés renfermant :

| | |
|---|---|
| **Produit de l'exemple 1** | 150 mg |
| Excipient q.s.p | 1 g |
| Détail de l'excipient : amidon, talc, stéarate de magnésium | |
| **Produit de l'exemple 2** | 150 mg |
| Excipient q.s.p | 1 g |
| Détail de l'excipient : amidon, talc, stéarate de magnésium | |
| **Produit de l'exemple 3** | 150 mg |
| Excipient q.s.p | 1 g |
| Détail de l'excipient : amidon, talc, stéarate de magnésium | |

### ETUDE PHARMACOLOGIQUE DES PRODUITS DE L'INVENTION

### Méthode des dilutions en milieu liquide

On prépare une série de tubes dans lesquels on répartit une même quantité de milieu nutritif stérile. On distribue dans chaque tube des quantités croissantes du produit à étudier, puis chaque tube est ensemencé avec une souche bactérienne. Après incubation de vingt-quatre heures à l'étuve à 37°C, l'inhibition de la croissance est appréciée par transillumination de ce qui permet de déterminer les concentrations minimales inhibitrices (C.M.I.) exprimées en microgrammes/cm³.

Les résultats suivants ont été obtenus :

| Souches bactériennes à GRAM⁺ | | | | | | | | |
|---|---|---|---|---|---|---|---|---|
| Produits | Ex 1 | Ex.2 | Ex.3 | Ex.28 | Ex.30 | Ex.31 | Ex.33 | Ex.34 |
| Staphylococcus aureus 011UC4 | 0,04 | 0,04 | 0,08 | 0,04 | 0,04 | 0,08 | 0,04 | 0,08 |
| Staphylococcus aureus 011G025I | 0,08 | 0,15 | 0,15 | 0,15 | 0,08 | 0,15 | 0,08 | 0,6 |
| Staphylococcus epidermidis 012GO11I | 0,08 | 0,04 | 0,15 | 0,04 | 0,4 | 0,08 | 0,04 | - |
| Streptococcus pyogenes groupe A 02A1UC1 | 0,04 | ≤0,02 | 0,04 | ≤ 0,02 | ≤ 0,02 | ≤ 0,02 | ≤ 0,02 | ≤ 0,02 |
| Streptococcus agalactiae groupe B 02B1HT1 | ≤0,02 | ≤0,02 | ≤0,02 | ≤0,02 | ≤0,02 | ≤0,02 | ≤ 0,02 | ≤ 0,02 |
| Streptococcus faecalis groupe D 02D2UC1 | 0,04 | ≤0,02 | 0,04 | ≤0,02 | ≤0,02 | ≤0,02 | ≤0,02 | ≤ 0,02 |
| Streptococcus faecium groupe D 02D3HT1 | ≤0,02 | ≤0,02 | 0,04 | ≤0,02 | ≤0,02 | ≤0,02 | 0,3 | ≤0,02 |
| Streptococcus sp groupe G 02G0GR5 | 0,04 | ≤0,02 | 0,04 | ≤0,02 | ≤0,02 | ≤0,02 | ≤0,02 | ≤0,02 |
| Streptococcus mitis 02mitCB1 | ≤0,02 | ≤0,02 | ≤0,02 | ≤0,02 | ≤0,02 | ≤0,02 | ≤0,02 | ≤0,02 |
| Streptococcus mitis 02mitGR16I | ≤0,02 | 0,15 | 0,04 | ≤0,02 | ≤0,02 | ≤0,02 | ≤0,02 | ≤0,02 |
| Streptococcus agalactiae groupe B 02B1SJ1 | 0,08 | 0,08 | 0,04 | - | 0,08 | 0,04 | 0,04 | 0,08 |
| Streptococcus pneumoniae 030SJ5 | 0,04 | 0,04 | 0,15 | 0,04 | 0,15 | 0,15 | ≤0,02 | ≤0,02 |

De plus, les produits des exemples 1, 2 et 3 ont montré une activité intéressante sur les souches bactériennes à gram^{⊖} suivantes : Haemophilus Influenzae 351HT3, 351CB12, 351CA1 et 351GR6.

## Revendications

1. Les composés de formule (I) : 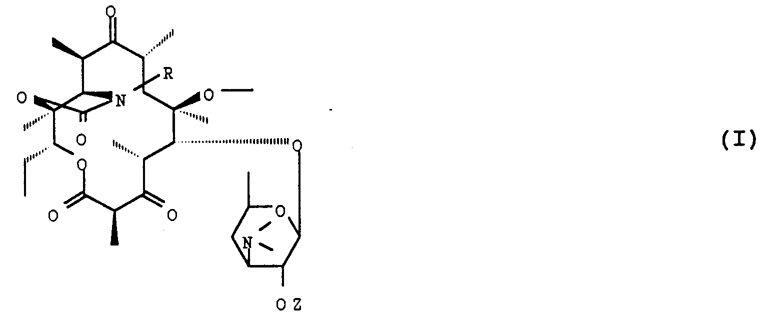 dans laquelle R représente un radical -(CH₂)ₙAr, dans lequel n représente le nombre 3, 4 ou 5 et Ar représente un radical hétérocyclique, portant éventuellement un ou plusieurs substituants, choisi dans le groupe des radicaux : 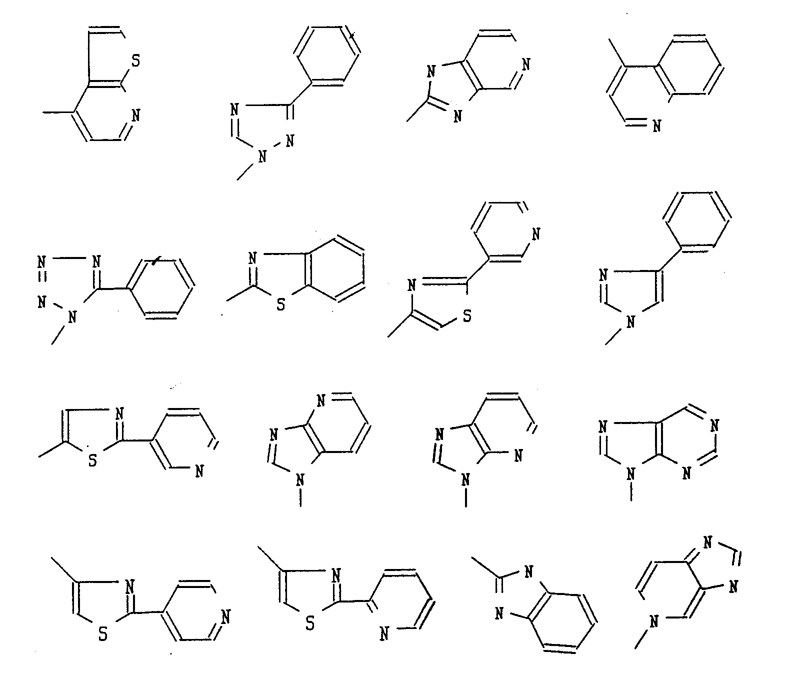 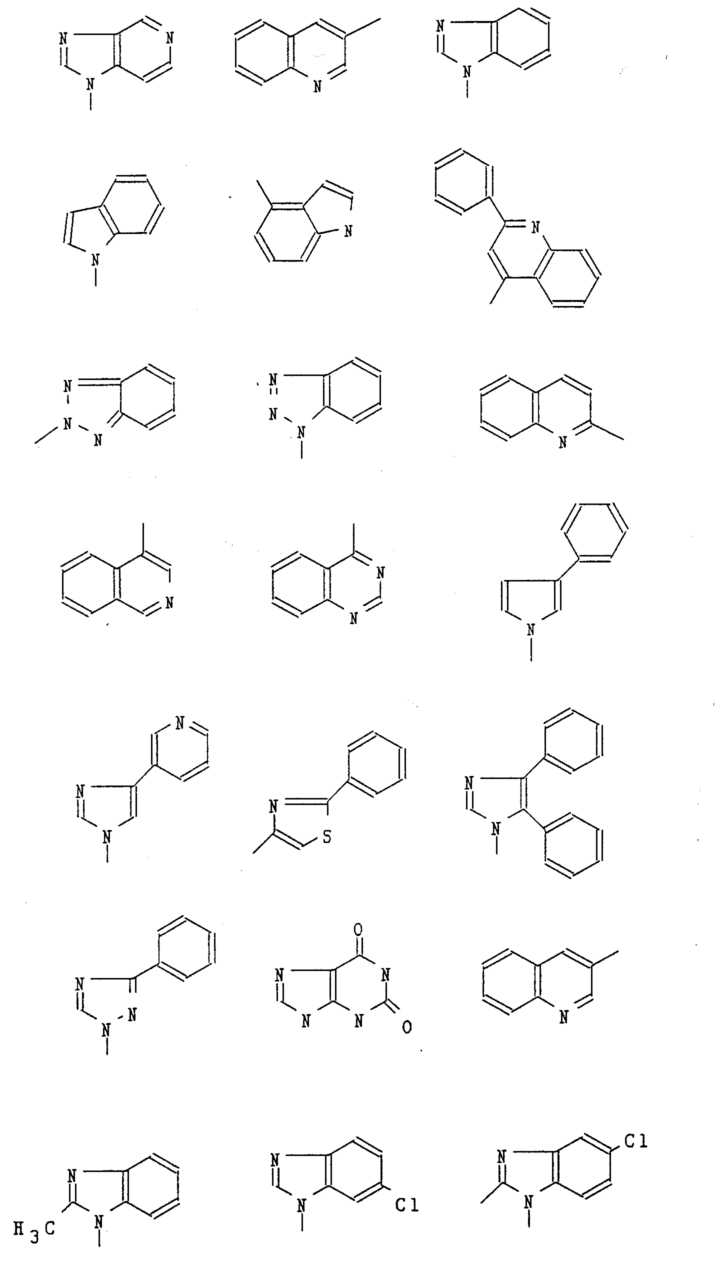 et Z représente un atome d'hydrogène ou le reste d'un acide, ainsi que leurs sels d'addition avec les acides, à l'exception des composés suivants :
11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(1H-indol-4-yl)butyl)imino) érythromycine
11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl ((4-(4-quinoléinyl) butyl) imino) érythromycine
11,12-didéoxy 3-de((2,6-didéoxy 3-C-méthyl 3-O-méthyl alpha-L-ribohexopyranosyl) oxy) 6-O-méthyl 3-oxo 12,11-(oxycarbonyl (4-(1H-benzimidazol-1-yl) butyl) imino) érythromycine.

2. Les composés de formule (I), tels que définis à la revendication 1, dans lesquels Z représente un atome d'hydrogène.

3. Les composés de formule (I), tels que définis à la revendication 1 ou 2, dans lesquels n représente le nombre 4.

4. Les composés de formule (I), tels que définis à la revendication 1, 2 ou 3, dans lesquels Ar représente un radical : 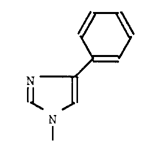 éventuellement substitué.

5. Les composés de formule (I), tels que définis à la revendication 1, 2 ou 3 dans lesquels R représente un radical : 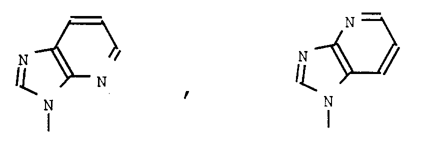 éventuellement substitué.

6. Les composés de formule (I), tels que définis à la revendication 1, 2 ou 3 dans lesquels Ar représente un radical : 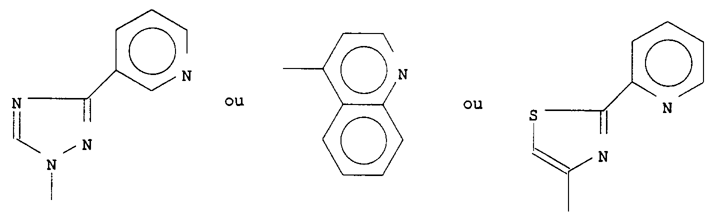 éventuellement substitué.

7. Les composés de formule (I), tels que définis à la revendication 1, 2 ou 3 dans lesquels Ar représente un radical : 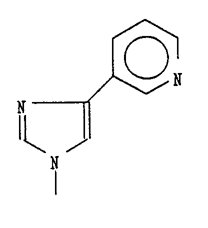 éventuellement substitué.

8. A titre de produits chimiques nouveaux les composés de formule (I), tels que définis à la revendication 1, dont les noms suivent :
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-phényl 1H-imidazol-1-yl) butyl) imino)) érythromycine,
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(3H-imidazo(4,5-b)pyridin-3-yl) butyl) imino)) érythromycine,
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(1H-imidazo(4,5-b)pyridin-1-yl) butyl) imino)) érythromycine,
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-(4-chlorophényl) 1H-imidazol-1-yl) butyl) imino)) érythromycine,
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-(2-méthoxyphényl) 1H-imidazol-1-yl) butyl) imino)) érythromycine,
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-(4-fluorophényl) 1H-imidazol-1-yl) butyl) imino)) érythromycine,
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(7-méthoxy 4-quinoléinyl) butyl) imino)) érythromycine,
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(2-(2-pyridinyl) 4-thiazolyl) butyl) imino)) érythromycine,
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(3-(3-pyridinyl) 1H-1,2,4-triazol-1-yl) butyl) imino)) érythromycine.

9. A titre de produit chimique nouveau le composé de formule (I), tel que défini à la revendication 1, dont le nom suit :
- 11,12-didéoxy 3-de((2,6-didéoxy-3-C-méthyl-3-O-méthyl-alpha-L-ribohexopyranosyl) oxy) 6-O-méthyl 3-oxo 12,11-(oxycarbonyl ((4-(4-(3-pyridinyl) 1H-imidazol-1-yl) butyl) imino)) érythromycine.

10. A titre de médicaments les composés de formule (I), tels que définis à l'une quelconque des revendications 1 à 7, ainsi que leurs sels d'addition avec les acides pharmaceutiquement acceptables.

11. A titre de médicaments les composés définis à la revendication 8, ainsi que leurs sels d'addition avec les acides pharmaceutiquement acceptables.

12. A titre de médicaments le composé défini à la revendication 9, ainsi que ses sels d'addition avec les acides pharmaceutiquement acceptables.

13. Compositions pharmaceutiques renfermant à titre de principe actif au moins un médicament selon l'une quelconque des revendications 10 à 12.

14. Procédé de préparation des composés de formule (I), tels que définis à la revendication 1, caractérisé en ce que l'on soumet un composé de formule (II) : 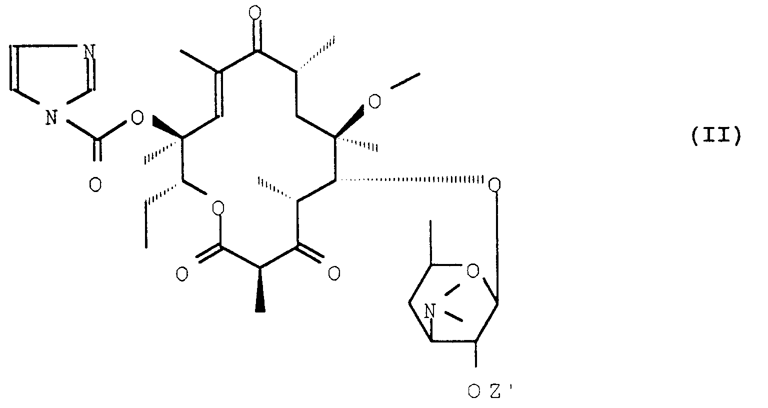 dans laquelle Z' représente le reste d'un acide, à l'action d'un composé de formule (III) :
RNH₂ (III)
dans laquelle R est défini comme à la revendication 1, pour obtenir le composé de formule (I_{A}) : 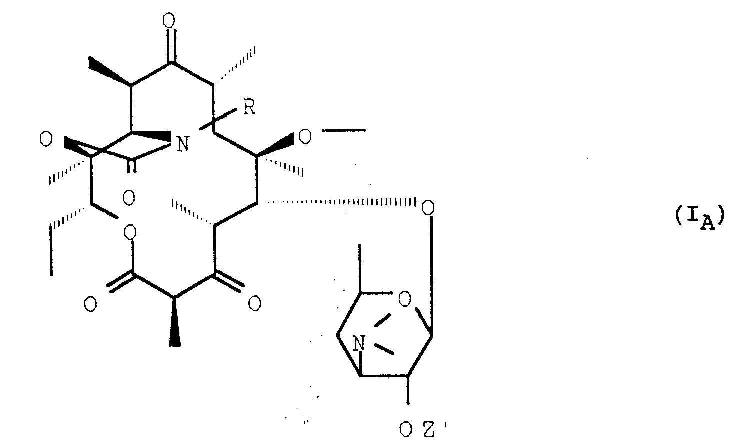 dans lesquels R et Z' conservent leur signification précédente, puis soumet le cas échéant, le composé de formule (I_{A}) à l'action d'un agent de libération de la fonction hydroxyle en 2' et/ou le cas échéant, à l'action d'un acide pour en former le sel.

15. Les composés de formule (III) tels que définis à la revendication 14.

16. Les amines de formule (III) telles que définies à la revendication 15, dont les noms suivent :
- la 4-phényl-1H-imidazole 1-butanamine,
- la 3H-imidazo(4,5-b)-pyridine 3-butanamine,
- la 1H-imidazo(4,5-b)-pyridine 3-butanamine,
- la 2-phényl-4-quinoleinebutanamine,
- la 1H-benzotriazole 1-butanamine,
- la 2H-benzotriazole 2-butanamine,
- la 1-méthyl 1H-imidazo(4,5-c)-pyridine 2-butanamine,
- la 3-méthyl 3H-imidazo(4,5-c)-pyridine 2-butanamine,
- la 5-chloro 1H-benzimidazole 1-butanamine,
- la 7-méthoxy 4-quinolèinebutanamine,
- la 1H-imidazo(4,5-c) pyridine 1-butanamine,
- la 9H-purine 9-butanamine,
- la 1-méthyl 1H-indole 4-butanamine,
- la 3-phényl 1H-1,2,4-triazole 1-butanamine (chlorhydrate),
- la 5-phényl 1H-tétrazole 1-butanamine (chlorhydrate),
- la 2-benzothiazolebutanamine,
- la 4-(thiéno(2,3-b) pyridine 4-yl butanamine,
- la 5,6-diméthyl 1H-benzimidazole 1-butanamine,
- la 3-quinoléine butanamine,
- la 2-quinoléine butanamine,
- la 5H-imidazo [4,5-c] pyridine 5-butanamine,
- la 1-méthyl 1H-benzimidazol 2-butanamine,
- la 6-chloro 1H-benzimidazol 2-butanamine,
- la 2-méthyl 1H-benzimidazol 2-butanamine,
- la 4-(4-chlorophényl) 1H-imidazol 1-butanamine,
- la 2-(3-pyridinyl) thiazol 5-butanamine,
- la 7-méthoxyquinoléine 4-butanamine,
- la 4-(4-fluorophényl) 1H-imidazol 1-butanamine,
- la 4-(2-méthoxyphényl) 1H-imidazol 1-butanamine,
- la 3-(3-pyridinyl) 1H 1,2,4-triazol 1-butanamine,
- la 4-(3-pyridinyl) 1H-imidazol 1-butanamine,
- la 2-(2-pyridinyl) thiazol 4-butanamine,
- la quinazoline 4-butanamine,
- la 4,5-diphényl 1H-imidazol 1-butanamine,
- la 4-(3-méthoxyphényl) 1H-imidazol 1-butanamine,
- la 4-(4-(trifluorométhoxy) phényl) 1H-imidazol 1-butanamine,
- la 1,2,3,6-tétrahydro 1,3-diméthyl 2,6-dioxo 7H-purine 7-butanamine,
- la 2-(4-pyridinyl) thiazol 4-butanamine,
- la 1H-indol 1-butanamine,
- la 2-(3-pyridinyl) thiazol 4-butanamine
ainsi que leurs sels d'addition avec les acides.

## Claims

1. The compounds of formula (I): 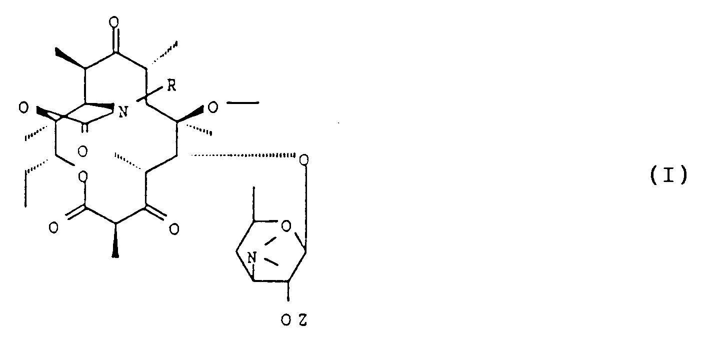 in which R represents a -(CH₂)ₙAr radical, in which n represents the number 3, 4 or 5 and Ar represents a heterocyclic radical, optionally carrying one or more substituents, chosen from the following group of radicals: 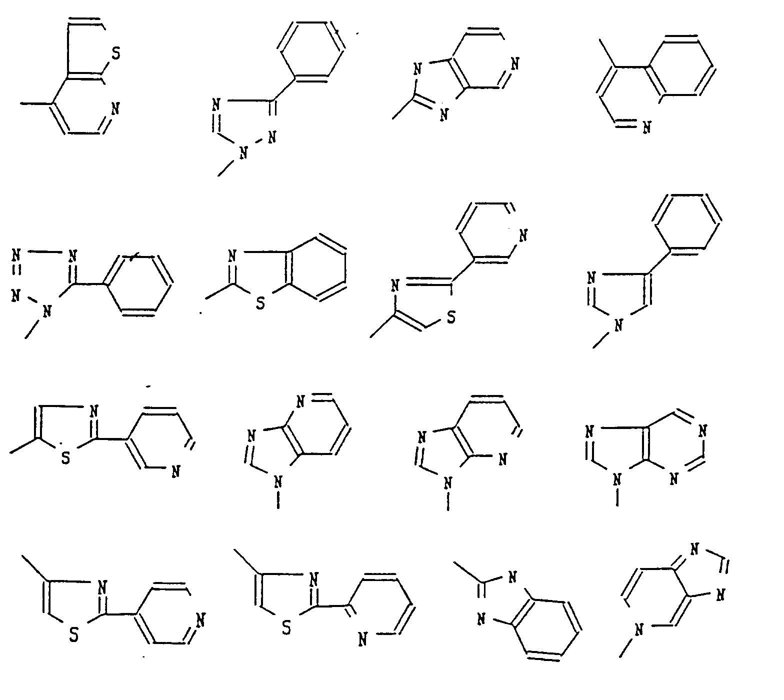 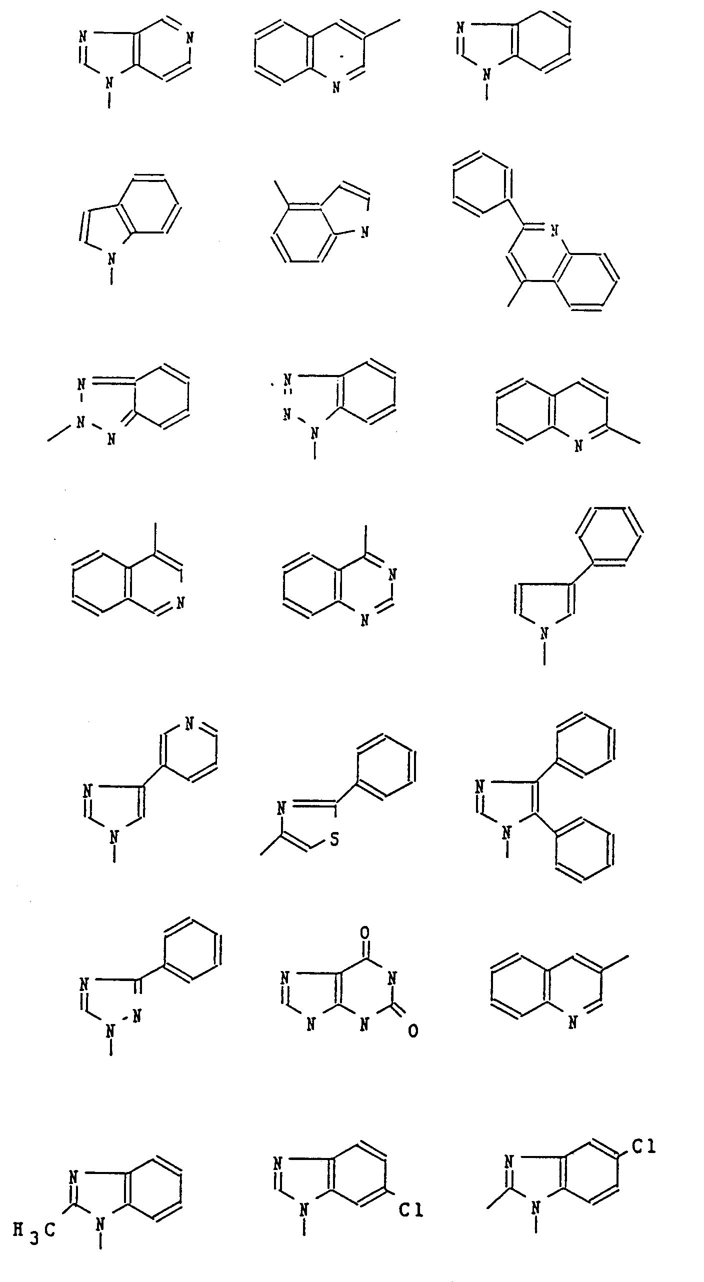 and Z represents a hydrogen atom or an acid remainder, as well as their addition salts with acids, except for the following compounds:
11,12-dideoxy 3-de((2,6-dideoxy 3-C-methyl 3-O-methyl alpha-L-ribohexopyranosyl) oxy) 6-O-methyl 3-oxo 12,11-(oxycarbonyl ((4-(1H-indol-4-yl)butyl)imino) erythromycin
11,12-dideoxy 3-de((2,6-dideoxy 3-C-methyl 3-O-methyl alpha-L-ribohexopyranosyl) oxy) 6-O-methyl 3-oxo 12,17-(oxycarbonyl ((4-(4-quinolinyl) butyl) imino) erythromycin
11,12-dideoxy 3-de((2,6-dideoxy 3-C-methyl 3-O-methyl alpha-L-ribohexopyranosyl) oxy) 6-O-methyl 3-oxo 12,11-(oxycarbonyl (4-(1H-benzimidazol-1-yl) butyl) imino) erythromycin.

2. The compounds of formula (I), as defined in claim 1, in which Z represents a hydrogen atom.

3. The compounds of formula (I), as defined in claim 1 or 2, in which n represents the number 4.

4. The compounds of formula (I), as defined in claim 1, 2 or 3, in which Ar represents a radical: 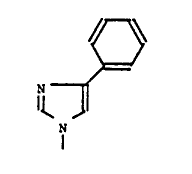 optionally substituted.

5. The compounds of formula (I), as defined in claim 1, 2 or 3, in which Ar represents a radical: 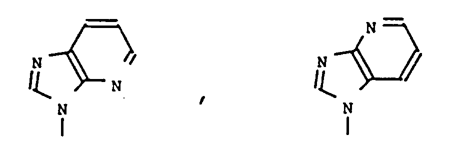 optionally substituted.

6. The compounds of formula (I), as defined in claim 1, 2 or 3 in which Ar represents a radical: 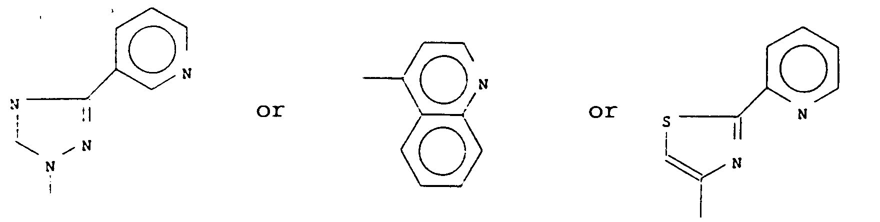 optionally substituted.

7. The compounds of formula (I), as defined in claim 1, 2 or 3 in which Ar represents a radical: 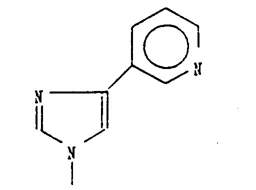 optionally substituted.

8. As new chemical products, the compounds of formula (I), as defined in claim 1, the names of which follow:
- 11,12-dideoxy 3-de((2,6-dideoxy-3-C-methyl-3-O-methyl-alpha-L-ribohexopyranosyl) oxy) 6-O-methyl 3-oxo 12,11-(oxycarbonyl ((4-(4-phenyl 1H-imidazol-1-yl) butyl) imino)) erythromycin,
- 11,12-dideoxy 3-de((2,6-dideoxy-3-C-methyl-3-O-methyl-alpha-L-ribohexopyranosyl) oxy) 6-O-methyl 3-oxo 12,11-(oxycarbonyl ((4-(3H-imidazo(4,5-b)pyridin-3-yl) butyl) imino)) erythromycin,
- 11,12-dideoxy 3-de((2,6-dideoxy-3-C-methyl-3-O-methyl-alpha-L-ribohexopyranosyl) oxy) 6-O-methyl 3-oxo 12,11-(oxycarbonyl ((4-(1H-imidazo(4,5-b)pyridin-1-yl) butyl) imino)) erythromycin,
- 11,12-dideoxy 3-de((2,6-dideoxy-3-C-methyl-3-O-methyl-alpha-L-ribohexopyranosyl) oxy) 6-O-methyl 3-oxo 12,11-(oxycarbonyl ((4-(4-(4-chlorophenyl) 1H-imidazol-1-yl) butyl) imino)) erythromycin,
- 11,12-dideoxy 3-de((2,6-dideoxy-3-C-methyl-3-O-methyl-alpha-L-ribohexopyranosyl) oxy) 6-O-methyl 3-oxo 12,11-(oxycarbonyl ((4-(4-(2-methoxyphenyl) 1H-imidazol-1-yl) butyl) imino)) erythromycin,
- 11,12-dideoxy 3-de((2,6-dideoxy-3-C-methyl-3-O-methyl-alpha-L-ribohexopyranosyl) oxy) 6-O-methyl 3-oxo 12,11-(oxycarbonyl ((4-(4-(4-fluorophenyl) 1H-imidazol-1-yl) butyl) imino)) erythromycin,
- 11,12-dideoxy 3-de((2,6-dideoxy-3-C-methyl-3-O-methyl-alpha-L-ribohexopyranosyl) oxy) 6-O-methyl 3-oxo 12,11-(oxycarbonyl ((4-(7-methoxy 4-quinolinyl) butyl) imino)) erythromycin,
- 11,12-dideoxy 3-de((2,6-dideoxy-3-C-methyl-3-O-methyl-alpha-L-rihohexopyranosyl) oxy) 6-O-methyl 3-oxo 12,11-(oxycarbonyl ((4-(2-(2-pyridinyl) 4-thiazolyl) butyl) imino)) erythromycin,
- 11,12-dideoxy 3-de((2,6-dideoxy-3-C-methyl-3-O-methyl-alpha-L-ribohexopyranosyl) oxy) 6-O-methyl 3-oxo 12,11-(oxycarbonyl ((4-(3-(3-pyridinyl) 1H-1,2,4-triazol-1-yl) butyl) imino)) erythromycin.

9. As a new chemical product, the compound of formula (I), as defined in claim 1, the name of which follows:
- 11,12-dideoxy 3-de((2,6-dideoxy-3-C-methyl-3-O-methyl-alpha-L-ribohexopyranosyl) oxy) 6-O-methyl 3-oxo 12,11-(oxycarbonyl ((4-(4-(3-pyridinyl) 1H-imidazol-1-yl) butyl) imino)) erythromycin.

10. As medicaments, the compounds of formula (I), as defined in any one of claims 1 to 7, as well as their addition salts with pharmaceutically acceptable acids.

11. As medicaments, the compounds defined in claim 8, as well as their addition salts with pharmaceutically acceptable acids.

12. As medicaments, the compound defined in claim 9, as well as its addition salts with pharmaceutically acceptable acids.

13. Pharmaceutical compositions containing as active ingredient at least one medicament according to any one of claims 10 to 12.

14. Preparation process for the compounds of formula (I), as defined in claim 1, characterized in that a compound of formula (II): 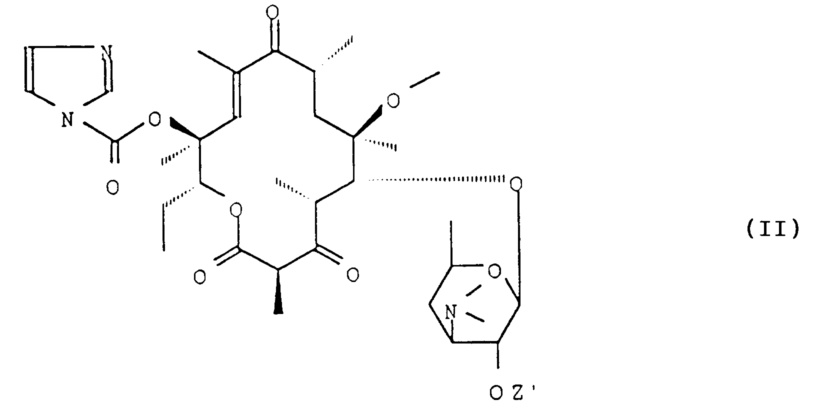 in which Z' represents an acid remainder, is subjected to the action of a compound of formula (III):
RNH₂ (III)
in which R is defined as in claim 1, in order to obtain the compound of formula (I_{A}): 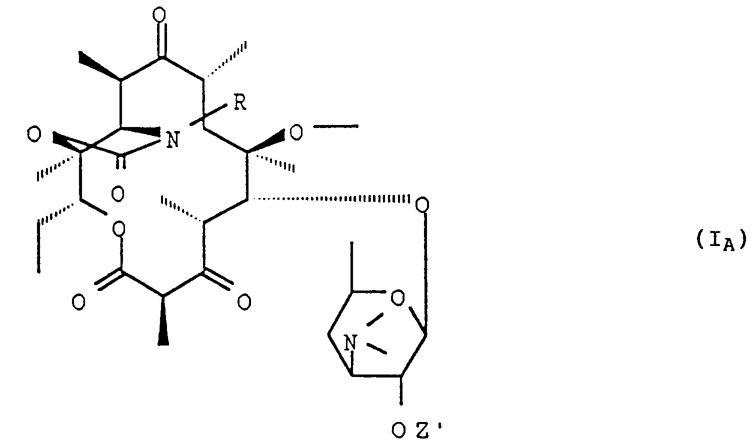 in which R and Z' keep their previous meaning, then if appropriate the compound of formula (I_{A}) is subjected to the action of an agent releasing the hydroxyl function in position 2' and/or if appropriate, to the action of an acid in order to form the salt.

15. The compounds of formula (III) as defined in claim 14.

16. The amines of formula (III) as defined in claim 15, the names of which follow:
- 4-phenyl-1H-imidazole 1-butanamine,
- 3H-imidazo(4,5-b)-pyridine 3-butanamine,
- 1H-imidazo(4,5-b)-pyridine 3-butanamine,
- 2-phenyl-4-quinolinebutanamine,
- 1H-benzotriazole 1-butanamine,
- 2H-benzotriazole 2-butanamine,
- 1-methyl 1H-imidazo(4,5-c)-pyridine 2-butanamine,
- 3-methyl 3H-imidazo(4,5-c)-pyridine 2-butanamine,
- 5-chloro 1H-benzimidazole 1-butanamine,
- 7-methoxy 4-quinolinebutanamine,
- 1H-imidazo(4,5-c) pyridine 1-butanamine,
- 9H-purine 9-butanamine,
- 1-methyl 1H-indole 4-butanamine,
- 3-phenyl 1H-1,2,4-triazole 1-butanamine (hydrochloride),
- 5-phenyl 1H-tetrazole 1-butanamine (hydrochloride),
- 2-benzothiozolebutanamine,
- 4-(thieno(2,3-b) pyridine 4-yl butanamine,
- 5,6-dimethyl 1H-benzimidazole 1-butanamine,
- 3-quinoline butanamine,
- 2-quinoline butanamine,
- 5H-imidazo [4,5-c] pyridine 5-butanamine,
- 1-methyl 1H-benzimidazol 2-butanamine,
- 6-chloro 1H-benzimidazol 2-butanamine,
- 2-methyl 1H-benzimidazol 2-butanamine,
- 4-(4-chlorophenyl) 1H-imidazol 1-butanamine,
- 2-(3-pyridinyl) thiazol 5-butanamine,
- 7-methoxyquinoline 4-butanamine,
- 4-(4-fluorophenyl) 1H-imidazol 1-butanamine,
- 4-(2-methoxyphenyl) 1H-imidazol 1-butanamine,
- 3-(3-pyridinyl) 1H 1,2,4-triazol 1-butanamine,
- 4-(3-pyridinyl) 1H-imidazol 1-butanamine,
- 2-(2-pyridinyl) thiazol 4-butanamine,
- 2-phenylthiazol 4-butanamine,
- 4-(4-methoxyphenyl) 1H-imidazol 1-butanamine,
- isoquinoline 4-butanamine,
- quinazoline 4-butanamine,
- 4,5-diphenyl 1H-imidazol 1-butanamine,
- 4-(3-methoxyphenyl) 1H-imidazol 1-butanamine,
- 4-(4-(trifluoromethoxy) phenyl) 1H-imidazol 1-butanamine,
- 1,2,3,6-tetrahydro 1,3-dimethyl 2,6-dioxo 7H-purine 7-butanamine,
- 2-(4-pyridinyl) thiazol 4-butanamine,
- 1H-indol 1-butanamine,
- 2-(3-pyridinyl) thiazol 4-butanamine
as well as their addition salts with acids.

## Patentansprüche

1. Die Verbindungen der Formel (I): 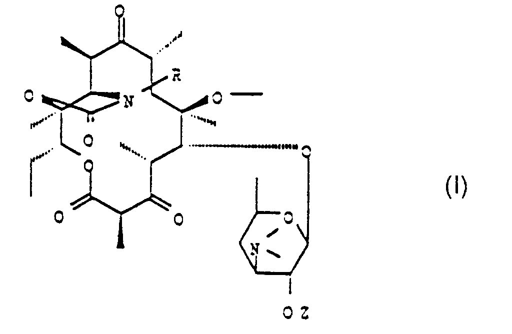 in der R einen -(CH₂)ₙAr-Rest darstellt, in dem n eine Zahl 3, 4 oder 5 darstellt und Ar einen heterocyclischen Rest darstellt, der gegebenenfalls einen oder mehrere Substituenten trägt, der aus der Gruppe der Reste: 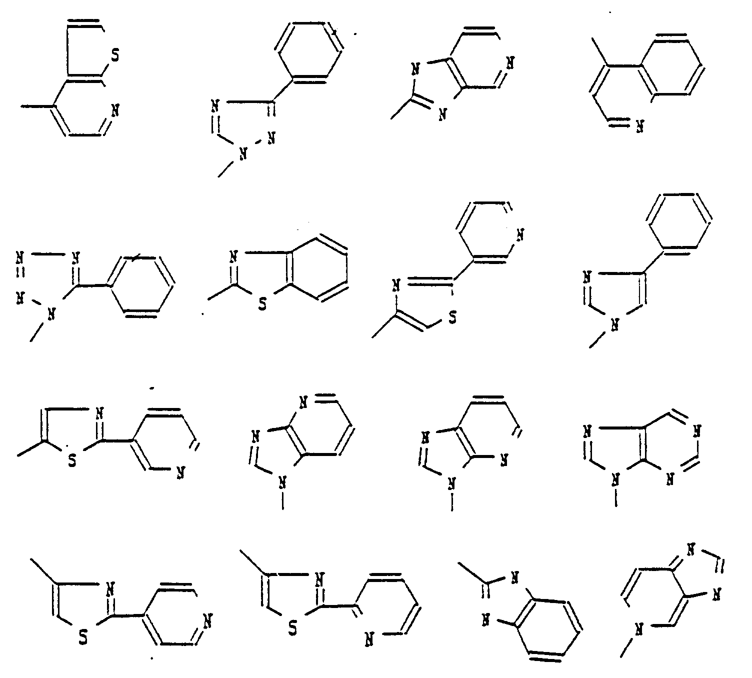 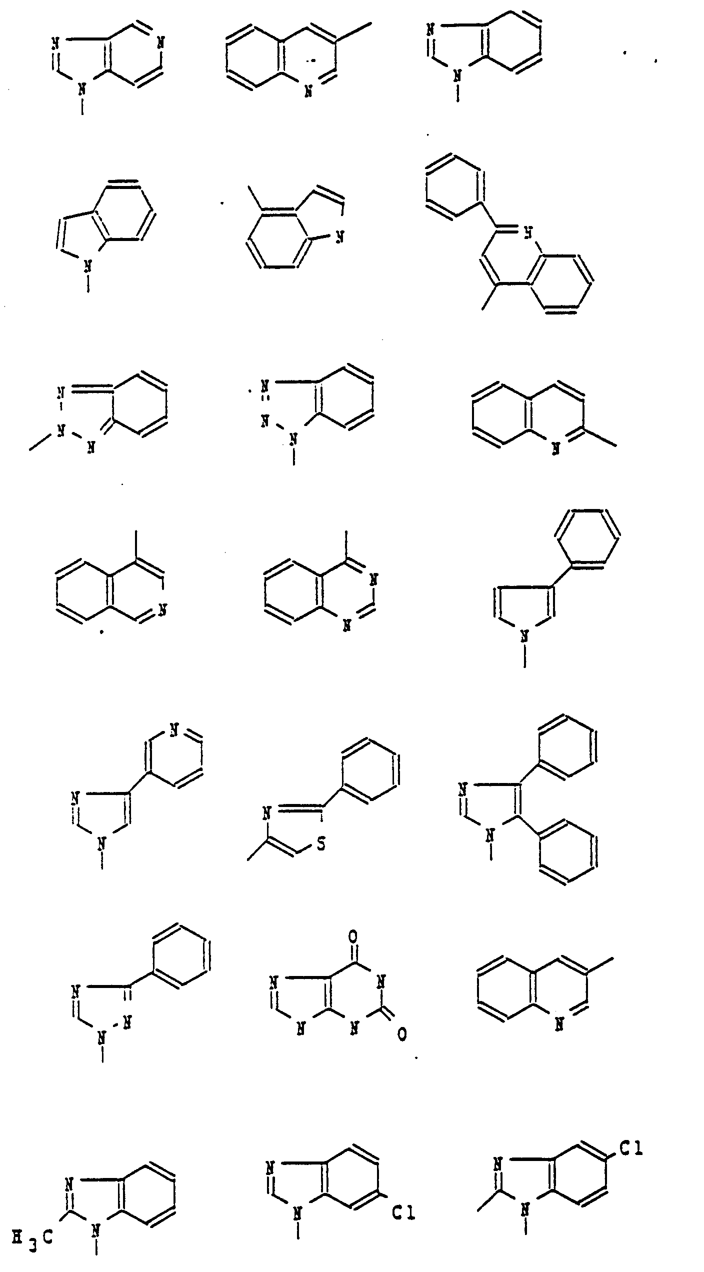 ausgewählt ist, und Z ein Wasserstoffatom oder den Rest einer Säure darstellt, sowie ihre Additionssalze mit den Säuren, mit Ausnahme der folgenden Verbindungen:
11,12-Dideoxy-3-de((2,6-dideoxy-3-C-methyl-3-O-methyl-alpha-L-ribohexopyranosyl)oxy)-6-O-methyl-3-oxo-12,11-(oxycarbonyl-((4-(1H-indol-4-yl)butyl)imino))erythromycin
11,12-Dideoxy-3-de((2,6-dideoxy-3-C-methyl-3-O-methyl-alpha-L-ribohexopyranosyl)oxy)-6-O-methyl-3-oxo-12,11-(oxycarbonyl-((4-(4-chinolinyl)butyl)imino))erythromycin
11,12-Dideoxy-3-de((2,6-dideoxy-3-C-methyl-3-O-methyl-alpha-L-ribohexopyranosyl)oxy)-6-O-methyl-3-oxo-12,11-(oxycarbonyl-(4-(1H-benzimidazol-1-yl)butyl)imino)erythromycin.

2. Die Verbindungen der Formel (I), wie in Anspruch 1 definiert, in denen Z ein Wasserstoffatom darstellt.

3. Die Verbindungen der Formel (I), wie in Anspruch 1 oder 2 definiert, in denen n die Zahl 4 darstellt.

4. Die Verbindungen der Formel (I), wie in Anspruch 1, 2 oder 3 definiert, in denen Ar einen Rest: 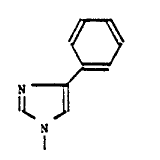 darstellt, der gegebenenfalls substituiert ist.

5. Die Verbindungen der Formel (I), wie in Anspruch 1, 2 oder 3 definiert, in denen R einen Rest: 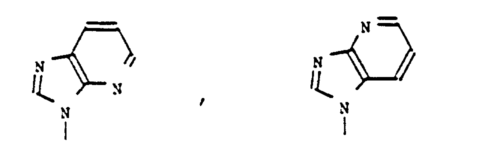 darstellt, der gegebenenfalls substituiert ist.

6. Die Verbindungen der Formel (I), wie in Anspruch 1, 2 oder 3 definiert, in denen Ar einen Rest: 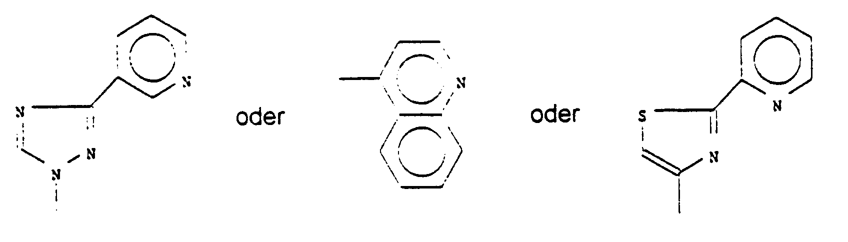 darstellt, der gegebenenfalls substituiert ist.

7. Die Verbindungen der Formel (I), wie in Anspruch 1, 2 oder 3 definiert, in denen Ar einen Rest: 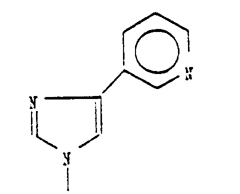 darstellt, der gegebenenfalls substituiert ist.

8. Als neue chemische Produkte die Verbindungen der Formel (I), wie in Anspruch 1 definiert, deren Namen folgen:
- 11,12-Dideoxy-3-de((2,6-dideoxy-3-C-methyl-3-O-methyl-alpha-L-ribohexopyranosyl)oxy)-6-O-methyl-3-oxo-12,11-(oxycarbonyl-((4-(4-phenyl-1H-imidazol-1-yl)butyl)imino))erythromycin,
- 11,12-Dideoxy-3-de((2,6-dideoxy-3-C-methyl-3-O-methyl-alpha-L-ribohexopyranosyl)oxy)-6-O-methyl-3-oxo-12,11-(oxycarbonyl-((4-(3H-imidazo(4,5-b)-pyridin-3-yl)butyl)imino))erythromycin,
- 11,12-Dideoxy-3-de((2,6-dideoxy-3-C-methyl-3-O-methyl-alpha-L-ribohexopyranosyl)oxy)-6-O-methyl-3-oxo-12,11-(oxycarbonyl-((4-(1H-imidazo(4,5-b)-pyridin-1-yl)butyl)imino))erythromycin,
- 11,12-Dideoxy-3-de((2,6-dideoxy-3-C-methyl-3-O-methyl-alpha-L-ribohexopyranosyl)oxy)-6-O-methyl-3-oxo-12,11-(oxycarbonyl-((4-(4-(4-chlorphenyl)-1H-imidazol-1-yl)butyl)imino))erythromycin,
- 11,12-Dideoxy-3-de((2,6-dideoxy-3-C-methyl-3-O-methyl-alpha-L-ribohexopyranosyl)oxy)-6-O-methyl-3-oxo-12,11-(oxycarbonyl-((4-(4-(2-methoxyphenyl)-1H-imidazol-1-yl)butyl)imino))erythromycin,
- 11,12-Dideoxy-3-de((2,6-dideoxy-3-C-methyl-3-O-methyl-alpha-L-ribohexopyranosyl)oxy)-6-O-methyl-3-oxo-12,11-(oxycarbonyl-((4-(4-(4-fluorphenyl)-1H-imidazol-1-yl)butyl)imino))erythromycin,
- 11,12-Dideoxy-3-de((2,6-dideoxy-3-C-methyl-3-O-methyl-alpha-L-ribohexopyranosyl)oxy)-6-O-methyl-3-oxo-12,11-(oxycarbonyl-((4-(7-methoxy-4-chinolinyl)butyl)imino))erythromycin,
- 11,12-Dideoxy-3-de((2,6-dideoxy-3-C-methyl-3-O-methyl-alpha-L-ribohexopyranosyl)oxy)-6-O-methyl-3-oxo-12,11-(oxycarbonyl-((4-(2-(2-pyridinyl)-4-thiazolyl)butyl)imino))erythromycin,
- 11,12-Dideoxy-3-de((2,6-dideoxy-3-C-methyl-3-O-methyl-alpha-L-ribohexopyranosyl)oxy)-6-O-methyl-3-oxo-12,11-(oxycarbonyl-((4-(3-(3-pyridinyl)-1H-1,2,4-triazol-1-yl)butyl)imino))erythromycin.

9. Als neues chemisches Produkt die Verbindung der Formel (I), wie in Anspruch 1 definiert, deren Namen folgt:
- 11,12-Dideoxy-3-de((2,6-dideoxy-3-C-methyl-3-O-methyl-alpha-L-ribohexopyranosyl)oxy)-6-O-methyl-3-oxo-12,11-(oxycarbonyl-((4-(4-(3-pyridinyl)-1H-imidazol-1-yl)butyl)imino))erythromycin.

10. Als Arzneimittel die Verbindungen der Formel (I), wie in einem der Ansprüche 1 bis 7 definiert, sowie ihre Additionssalze mit den pharmazeutisch akzeptablen Säuren.

11. Als Arzneimittel die in Anspruch 8 definierten Verbindungen sowie ihre Additionssalze mit den pharmazeutisch akzeptablen Säuren.

12. Als Arzneimittel die in Anspruch 9 definierte Verbindung sowie ihre Additionssalze mit den pharmazeutisch akzeptablen Säuren.

13. Pharmazeutische Zusammensetzungen, die als Wirkstoff wenigstens ein Medikament gemäß einem der Ansprüche 10 bis 12 enthalten.

14. Verfahren zur Herstellung der Verbindungen der Formel (I), wie in Anspruch 1 definiert, dadurch gekennzeichnet, daß man eine Verbindung der Formel (II): 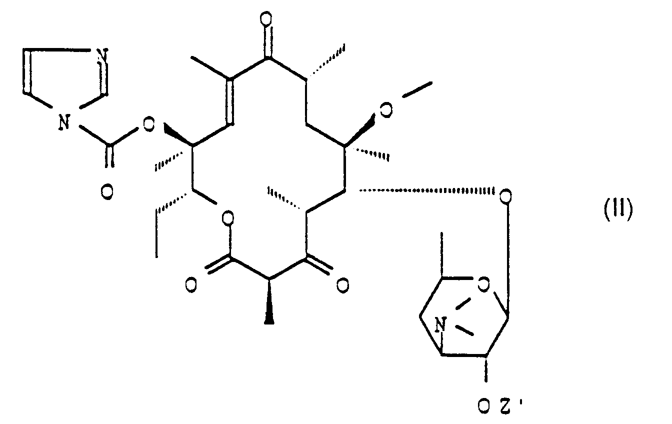 in der Z' den Rest einer Säure darstellt, der Einwirkung einer Verbindung der Formel (III):
RNH₂ (III)
in der R wie in Anspruch 1 definiert ist, unterzieht, um die Verbindung der Formel (I_{A}): 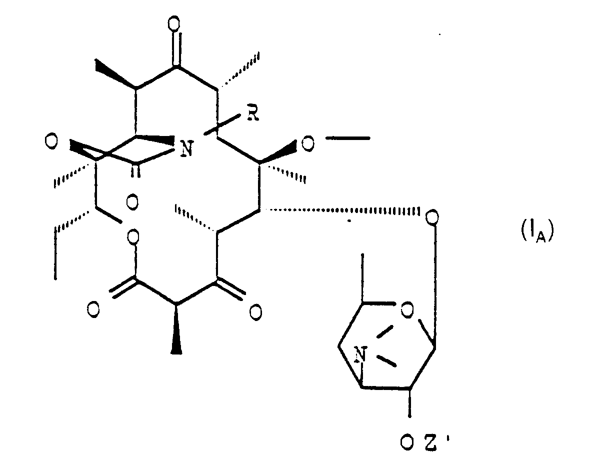 in der R und Z' ihre vorhergehende Bedeutung behalten, zu erhalten, dann gegebenenfalls die Verbindung der Formel (I_{A}) der Einwirkung eines Mittels zur Freisetzung der Hydroxyfunktion in 2' und/oder gegebenenfalls der Einwirkung einer Säure unterzieht, um daraus das Salz zu bilden.

15. Die Verbindungen der Formel (III), wie in Anspruch 14 definiert.

16. Die Amine der Formel (III), wie in Anspruch 15 definiert, deren Namen folgen:
- 4-Phenyl-1H-imidazol-1-butanamin,
- 3H-Imidazo(4,5-b)-pyridin-3-butanamin,
- 1H-Imidazo(4,5-b)-pyridin-3-butanamin,
- 2-Phenyl-4-chinolin-butanamin,
- 1H-Benzotriazol-1-butanamin,
- 2H-Benzotriazol-2-butanamin,
- 1-Methyl-1H-imidazo(4,5-c)-pyridin-2-butanamin,
- 3-Methyl-3H-imidazo(4,5-c)-pyridin-2-butanamin,
- 5-Chlor-1H-benzimidazol-1-butanamin,
- 7-Methoxy-4-chinolin-butanamin,
- 1H-Imidazo(4,5-c)-pyridin-1-butanamin,
- 9H-Purin-9-butanamin,
- 1-Methyl-1H-indol-4-butanamin,
- 3-Phenyl-1H-1,2,4-triazol-1-butanamin (Chlorhydrat),
- 5-Phenyl-1H-tetrazol-1-butanamin (Chlorhydrat),
- 2-Benzothiazol-butanamin,
- 4-(Thieno(2,3-b)-pyridin-4-yl)-butanamin,
- 5,6-Dimethyl-1H-benzimidazol-1-butanamin,
- 3-Chinolin-butanamin,
- 2-Chinolin-butanamin,
- 5H-Imidazo(4,5-c)-pyridin-5-butanamin,
- 1-Methyl-1H-benzimidazol-2-butanamin,
- 6-Chlor-1H-benzimidazol-2-butanamin,
- 2-Methyl-1H-benzimidazol-2-butanamin,
- 4-(4-Chlorphenyl)-1H-imidazol-1-butanamin,
- 2-(3-Pyridinyl)-thiazol-5-butanamin,
- 7-Methoxychinolin-4-butanamin,
- 4-(4-Fluorphenyl)-1H-imidazol-1-butanamin,
- 4-(2-Methoxyphenyl)-1H-imidazol-1-butanamin,
- 3-(3-Pyridinyl)-1H-1,2,4-triazol-1-butanamin,
- 4-(3-Pyridinyl)-1H-imidazol-1-butanamin,
- 2-(2-Pyridinyl)-thiazol-4-butanamin,
- 2-Phenylthiazol-4-butanamin,
- 4-(4-Methoxyphenyl)-1H-imidazol-1-butanamin,
- Isochinolin-4-butanamin,
- Chinazolin-4-butanamin,
- 4,5-Diphenyl-1H-imidazol-1-butanamin,
- 4-(3-Methoxyphenyl)-1H-imidazol-1-butanamin,
- 4-(4-(Trifluormethoxy)phenyl)-1H-imidazol-1-butanamin,
- 1,2,3,6-Tetrahydro-1,3-dimethyl-2,6-dioxo-7H-purin-7-butanamin,
- 2-(4-Pyridinyl)-thiazol-4-butanamin,
- 1H-Indol-1-butanamin,
- 2-(3-Pyridinyl)-thiazol-4-butanamin,
sowie ihre Additionssalze mit den Säuren.
